(19) Europäisches Patentamt
European Patent Office
Office européen des brevets

(11) EP 1 963 272 B1

(12) # EUROPÄISCHE PATENTSCHRIFT

(45) Veröffentlichungstag und Bekanntmachung des Hinweises auf die Patenterteilung:
**20.03.2013 Patentblatt 2013/12**

(21) Anmeldenummer: **06818521.4**

(22) Anmeldetag: **14.11.2006**

(51) Int Cl.:
***C07D 215/38*** (2006.01) ***C07D 401/12*** (2006.01)
***A61K 31/47*** (2006.01) ***A61K 31/4709*** (2006.01)
***A61P 35/00*** (2006.01)

(86) Internationale Anmeldenummer:
**PCT/EP2006/010902**

(87) Internationale Veröffentlichungsnummer:
**WO 2007/068316 (21.06.2007 Gazette 2007/25)**

(54) **HYDROXYCHINOLINDERIVATE**
HYDROXYQUINOLINE DERIVATIVES
DERIVES D HYDROXYQUINOLEINE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **13.12.2005 DE 102005059479**

(43) Veröffentlichungstag der Anmeldung:
**03.09.2008 Patentblatt 2008/36**

(73) Patentinhaber: **Merck Patent GmbH**
**64293 Darmstadt (DE)**

(72) Erfinder:
- **SCHADT, Oliver**
**63517 Rodenbach (DE)**
- **DORSCH, Dieter**
**64372 Ober-Ramstadt (DE)**
- **FITTSCHEN, Claus**
**64407 Fraenkisch-Crumbach (DE)**
- **GRELL, Matthias**
**64291 Darmstadt (DE)**

(56) Entgegenhaltungen:
**WO-A-02/44166 WO-A-03/068749**
**WO-A-03/080578 US-A- 6 030 983**

- **DATABASE CAPLUS [Online] CHEMICAL ABSTRACTS SERVICE, COLUMBUS, OHIO, US; ALBRECHT, MARKUS ET AL: "Inter- and intramolecular hydrogen bonding in amide- and urea-substituted 8-hydroxyquinoline derivatives" XP002412788 gefunden im STN Database accession no. 2002:6266 & TETRAHEDRON , 58(3), 561-567 CODEN: TETRAB; ISSN: 0040-4020, 2002,**
- **DATABASE CAPLUS [Online] CHEMICAL ABSTRACTS SERVICE, COLUMBUS, OHIO, US; ALBRECHT, MARKUS ET AL: "Solid-state structures of amide-substituted 8-hydroxyquinoline derivatives" XP002412789 gefunden im STN Database accession no. 2000: 97384 & TETRAHEDRON , 56(4), 591-594 CODEN: TETRAB; ISSN: 0040-4020, 2000,**
- **DATABASE CAPLUS [Online] CHEMICAL ABSTRACTS SERVICE, COLUMBUS, OHIO, US; KATO, KATSUNORI ET AL: "Metal complexes of quinolinedione-aniline adducts for optical recording" XP002412790 gefunden im STN Database accession no. 1991:666981 & JP 03 103476 A (KONICA CO., JAPAN) 30. April 1991 (1991-04-30)**
- **DATABASE CAPLUS [Online] CHEMICAL ABSTRACTS SERVICE, COLUMBUS, OHIO, US; WATANABE, KATSUYUKI ET AL: "Infrared-absorbing-image-forming color photographic material" XP002412791 gefunden im STN Database accession no. 1991:237515 & JP 02 187754 A (FUJI PHOTO FILM CO., LTD., JAPAN) 23. Juli 1990 (1990-07-23)**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



(Forts. nächste Seite)

- DATABASE CAPLUS [Online] CHEMICAL ABSTRACTS SERVICE, COLUMBUS, OHIO, US; MATSUMURA, KONOMU ET AL: "Amebicidal 8-quinolinol compounds" XP002412792 gefunden im STN Database accession no. 1961:8120 & JOURNAL OF ORGANIC CHEMISTRY , 25, 853-4 CODEN: JOCEAH; ISSN: 0022-3263, 1960,
- DATABASE CAPLUS [Online] CHEMICAL ABSTRACTS SERVICE, COLUMBUS, OHIO, US; MATSUMURA, KONOMU ET AL: "Synthesis of quinoline derivatives. VI. Preparation of certain acylamino derivatives of 8-hydroxyquinoline" XP002412793 gefunden im STN Database accession no. 1931:8710 & JOURNAL OF THE AMERICAN CHEMICAL SOCIETY , 53, 177-9 CODEN: JACSAT; ISSN: 0002-7863, 1931,

## Beschreibung

Die Erfindung betrifft Verbindungen der Formel I

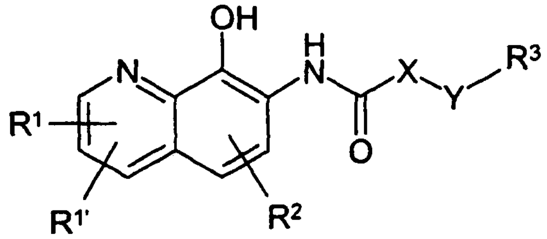

I

worin

$R^1$, $R^{1'}$, $R^2$ jeweils unabhängig voneinander H, OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA oder CN,

$R^3$ A, Ar, Het, $NR^4R^5$ oder $CHR^4R^5$,
wobei mindestens einer der Reste
$R^4$ oder $R^5$ $(CH_2)_nAr$ oder $(CH_2)_nHet$ bedeutet,

$R^4$, $R^5$ jeweils unabhängig voneinander H, A, $(CH_2)_nAr$ oder $(CH_2)_nHet$,

X fehlt, $CH_2$, $NR^6$ oder O,

Y fehlt oder Alkylen mit 1-3 C-Atomen,

$R^6$ H oder A,

A, A' jeweils unabhängig voneinander unverzweigtes oder verzweigtes Alkyl mit 1-10 C-Atomen,

worin 1-7 H-Atome durch F, Cl und/oder Br ersetzt sein

können,
Cycloalkyl mit 3-8 C-Atomen oder
Cycloalkylalkylen mit 4-10 C-Atomen,

Hal F, Cl, Br oder I,

Ar einen unsubstituierten oder ein-, zwei- oder dreifach durch OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA, COA, CHO oder CN substituierten gesättigten, ungesättigten oder aromatischen Carbocyclus mit 5-14 C-Atomen,

Het einen ein-, zwei- oder dreikemigen gesättigten, ungesättigten oder aromatischen Heterocyclus mit 1 bis 4 N-, O- und/oder S-Atomen, der unsubstituiert oder ein-, zwei- oder dreifach durch OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA, CHO, COA, CN, =S, =NH, =NA und/oder =O (Carbonylsauerstoff) substituiert sein kann,

n 0, 1 oder 2,

bedeuten,
sowie ihre pharmazeutisch verwendbaren Derivate, Solvate, Salze, Tautomere und Stereoisomere, einschließlich deren Mischungen in allen Verhältnissen, wobei die nachfolgenden Verbindungen ausgenommen sind
N-(8-Hydroxy-7-chinolyl)-benzamid, N-(8-Hydroxy-7-chinolyl)-octanamid,
2-(Acetylamino)-N-(8-hydroxy-7-chinolyl)-acetamid, N-(8-Hydroxy-7-chinolyl)-N'-phenyl-harnstoff,
N-(8-Hydroxy-7-chinolyl)-N'-octyl-harnstoff, N-(8-Hydroxy-7-chinolyl)-N'-(phenylmethyl)-harnstoff,
N-(8-Hydroxy-7-chinolyl)-acetamid, N-(5-Chlor-8-hydroxy-7-chinolyl)-2-methyl-propanamid,
4-[[[(5-Chlor-8-hydroxy-7-chinolyl)amino]carbonyl]amino]-N,N-dioctyl-benzolsulfonamid,
7-Acetamido-8-hydroxy-5-chinolin-carbonsäure, 7-Acetamido-5-methyl-8-chinolinol.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze und/oder Solvate bei guter Verträglichkeit sehr wertvolle pharmakologische Eigenschaften besitzen.

Insbesondere zeigen sie eine inhibierende Wirkung der Zellproliferation als Antagonisten oder Agonisten. Die erfindungsgemäßen Verbindungen können daher zur Bekämpfung und/oder Behandlung von Tumoren, Tumorwachstum

und/oder Tumormetastasen verwendet werden.
Die antiproliferative Wirkung kann in einem HCT116 Proliferationsassay getestet werden.

Andere Chinolinderivate zur Krebsbekämpfung sind beispielsweise aus US 6,030,983, aus US 5,712,289, aus US 5,646,150 oder WO 94/29308 bekannt.

Dementsprechend werden die erfindungsgemäßen Verbindungen oder ein pharmazeutisch unbedenkliches Salz davon für die Behandlung von Krebs verabreicht, einschließlich solider Karzinome, wie zum Beispiel Karzinome (z. B. der Lungen, des Pankreas, der Schilddrüse, der Harnblase oder des Kolons), myeloische Erkrankungen (z. B. myeloische Leukämie) oder Adenome (z. B. villöses Kolonadenom).
Zu den Tumoren zählen weiterhin die Monozytenleukämie, Hirn-, Urogenital-, Lymphsystem-, Magen-, Kehlkopf- und Lungenkarzinom, darunter Lungenadenokarzinom und kleinzelliges Lungenkarzinom, Bauchspeicheldrüsen- und/oder Brustkarzinom.

Die Verbindungen sind ferner nützlich bei der Behandlung der durch HIV-1 (Human Immunodeficiency Virus Typ 1) induzierten Immunschwäche.

Als krebsartige hyperproliferative Erkrankungen sind Himkrebs, Lungenkrebs, Plattenepithelkrebs, Blasenkrebs, Magenkrebs, Pankreaskrebs, Leberkrebs, Nierenkrebs, Kolorektalkrebs, Brustkrebs, Kopfkrebs, Halskrebs, Ösophaguskrebs, gynäkologischer Krebs, Schilddrüsenkrebs, Lymphome, chronische Leukämie und akute Leukämie anzusehen. Insbesondere krebsartiges Zellwachstum ist eine Erkrankung, die ein Ziel der vorliegenden Erfindung darstellt. Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Es kann gezeigt werden, dass die erfindungsgemäßen Verbindungen antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit erreicht, z. B. zur Verhinderung des Tumorwachstums. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

Gegenstand der Erfindung sind auch die optisch aktiven Formen (Stereoisomeren), Salze, die Enantiomeren,

die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:

verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.

Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Ansprüchen 1-13 sowie ihrer pharmazeutisch verwendbaren Salze, Solvate, Tautomeren und Stereoisomeren, dadurch gekennzeichnet, daß man

a) zur Herstellung von Verbindungen der Formel I, worin

X fehlt oder $CH_2$ bedeutet,

eine Verbindung der Formel II

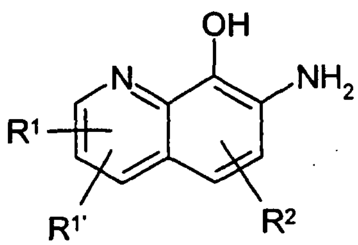

II

worin $R^1$, $R^{1'}$ und $R^2$ die in Anspruch 1 angegebenen Bedeutungen haben,
mit einer Verbindung der Formel III

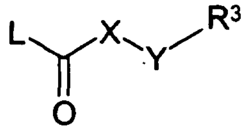

III ,

worin X fehlt oder $CH_2$ bedeutet,
Y und $R^3$ die in Anspruch 1 angegebenen Bedeutungen haben, und L Cl, Br, I oder eine freie oder reaktionsfähig funktionell abgewandelte OH-Gruppe bedeutet,
umsetzt,
oder
b) zur Herstellung von Verbindungen der Formel I, worin

X $NR^6$ bedeutet,

eine Verbindung der Formel II mit einer Verbindung der Formel IV

$O{=}C{=}N\text{-}Y\text{-}R^3$ IV

worin
Y und $R^3$ die in Anspruch 1 angebenen Bedeutungen haben, umsetzt, oder
c) daß man sie aus einem ihrer funktionellen Derivate durch Behandeln mit einem solvolysierenden oder hydrogenolysierenden Mittel in Freiheit setzt,
oder
d) zur Herstellung von Verbindungen der Formel I, worin

X NR$^6$ oder O bedeutet,

eine Verbindung der Formel V

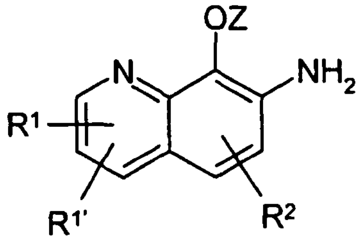

V

worin $R^1$, $R^{1'}$ und $R^2$ die in Anspruch 1 angegebenen Bedeutungen haben,
und Z eine Hydroxy-Schutzgruppe bedeutet,
mit einem Kupplungsreagenz ausgewählt aus der Gruppe

a) Isoproylidenchloroformiat,
b) p-Nitrophenylchloroformiat,
c) Diphosgen,
d) Triphosgen,

und einer Verbindung der Formel VI

H-X-Y-$R^3$ VI

worin X NR$^6$ oder O,
Y und $R^3$ die in Anspruch 1 angegebenen Bedeutungen haben,
umsetzt, und anschließend die Hydroxyschutzgruppe Z abspaltet,

und/oder
eine Base oder Säure der Formel I in eines ihrer Salze umwandelt.

Vor- und nachstehend haben die Reste X, Y, $R^1$, $R^{1'}$, $R^2$ und $R^3$ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
Cycloalkyl bedeutet vorzugsweise Cyclopropyl, Cyclobutyl, Cylopentyl, Cyclohexyl oder Cycloheptyl.
Cycloalkylalkylen bedeutet vorzugsweise Cyclopropylmethyl, Cyclobutylmethyl, Cylopentylmethyl, Cyclohexylmethyl oder Cycloheptylmethyl.
Alkylen ist vorzugsweise unverzweigt und bedeutet bevorzugt Methylen, Ethylen oder Propylen.

Ein gesättigter, ungesättigter oder aromatischer Carbocyclus mit 5-14 C-Atomen bedeutet vorzugsweise, Cyclopentyl, Cyclohexyl, Cycloheptyl, Phenyl, Naphthyl, Biphenyl oder Tetrahydronaphthyl.

Ar bedeutet vorzugsweise einen unsubstituierten oder ein-, zwei- oder dreifach durch OH, OA, Hal und/oder A substituierten gesättigten, ungesättigten oder aromatischen Carbocyclus mit 6-14 C-Atomen.

Ar bedeutet z.B. Phenyl, o-, m- oder p-Tolyl, o-, m- oder p-Ethylphenyl, o-, m- oder p-Propylphenyl, o-, m- oder

p-Isopropylphenyl, o-, m- oder p-tert.-Butylphenyl, o-, m- oder p-Trifluormethylphenyl, o-, m- oder p-Fluorphenyl, o-, m- oder p-Bromphenyl, o-, m- oder p-Chlorphenyl, o-, m- oder p-Hydroxyphenyl, o-, m- oder p-Methoxyphenyl, o-, m- oder p-Methylsulfonylphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Aminosulfonylphenyl, o-, m- oder p-Methylaminosulfonylphenyl, o-, m- oder p-Aminocarbonylphenyl, o-, m- oder p-Carboxyphenyl, o-, m- oder p-Methoxycarbonylphenyl, o-, m- oder p-Ethoxycarbonylphenyl, o-, m- oder p-Acetylphenyl, o-, m- oder p-Formylphenyl, o-, m- oder p-Cyanphenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3,4-, 2,3,5-, 2,3,6-, 2,4,6- oder 3,4,5-Trichlorphenyl, p-Iodphenyl, 4-Fluor-3-chlorphenyl, 2-Fluor-4-bromphenyl, 2,5-Difluor-4-bromphenyl oder 2,5-Dimethyl-4-chlorphenyl.

Ar bedeutet besonders bevorzugt unsubstituiertes oder ein-, zwei- oder dreifach durch OH, OA, Hal und/oder A substituiertes Phenyl, Naphthyl oder 1,2,3,4-Tetrahydronaphthalin.

Het bedeutet, ungeachtetet weiterer Substitutionen, z.B. 2- oder 3-Furyl, 2-oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1 ,2,3-Triazol-1-, -4- oder-5-yl, 1,2,4-Triazol-1-, -3- oder 5-yl, 1- oder 5-Tetrazolyl, 1,2,3-Oxadiazol-4- oder -5-yl, 1,2,4-Oxadiazol-3-oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7-oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Unsubstituiertes Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, - 3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, - 2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-, -2-, -3-, -4-, -5-, -6-, -7- oder -8-isochinolyl, 2-, 3-, 5-, 6-, 7- oder 8- 3,4-Dihydro-2H-benzo[1,4]-oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydrobenzofuranyl oder 2,3-Dihydro-2-oxo-furanyl.

Het bedeutet vorzugsweise einen einkernigen aromatischen Heterocyclus mit 1 bis 3 N-, O und/oder S-Atomen.
Het bedeutet ganz besonders bevorzugt Pyridyl, Pyrimidinyl, Thienyl oder Furyl.

$R^1$, $R^{1'}$ bedeuten vorzugsweise H.
$R^2$ bedeutet vorzugsweise H, OA oder Hal.
X bedeutet vorzugsweise fehlt, $CH_2$ oder NH.
Y fehlt vorzugsweise.
$R^3$ bedeutet vorzugsweise

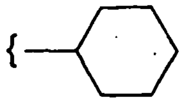 , 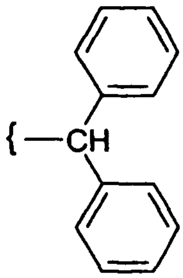 , 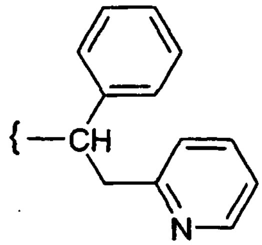 ,

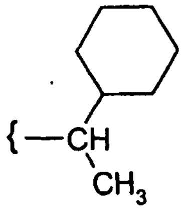 , 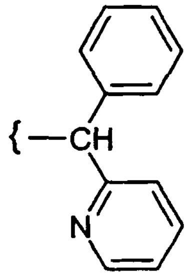 , 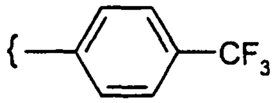 ,

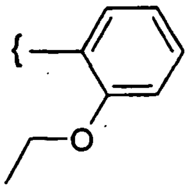 , 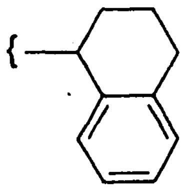 , 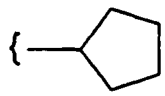 ,

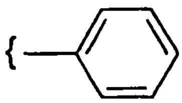 , 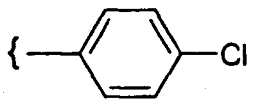 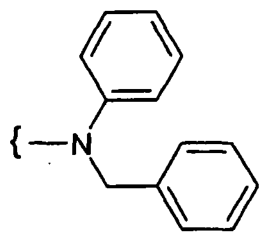 ,

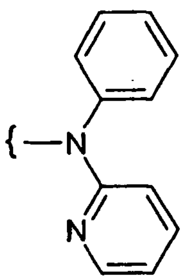 , oder 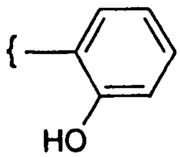 ,

Hal bedeutet vorzugsweise F, Cl oder Br, aber auch I, besonders bevorzugt F oder Cl.

Für die gesamte Erfindung gilt, daß sämtliche Reste, die mehrfach auftreten, gleich oder verschieden sein können, d.h. unabhängig voneinander sind.

Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Ik ausgedrückt werden, die der Formel I entsprechen und worin die nicht näher bezeichneten Reste die bei der Formel I angegebene Bedeutung haben, worin jedoch

in Ia $R^1$, $R^{1'}$ H bedeutet;
in Ib $R^2$ H, OA oder Hal bedeutet;

(fortgesetzt)

in Ic Ar einen unsubstituierten oder ein-, zwei- oder dreifach durch OH, OA, Hal und/oder A substituierten gesättigten, ungesättigten oder aromatischen Carbocyclus mit 5-14 C-Atomen bedeutet;

in Id Het einen einkernigen aromatischen Heterocyclus mit 1 bis 3 N-, O und/oder S-Atomen bedeutet;

in Ie X fehlt, $CH_2$ oder NH bedeutet;

in If Y fehlt bedeutet;

in Ig Ar unsubstituiertes oder ein-, zwei- oder dreifach durch OH, OA, Hal und/oder A substituiertes Phenyl, Naphthyl oder 1,2,3,4-Tetrahydronaphthalin bedeutet;

in Ih Het Pyridyl, Pyrimidinyl, Thienyl oder Furyl bedeutet;

in Ii $R^3$

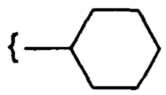 , 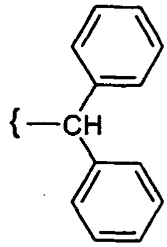 , 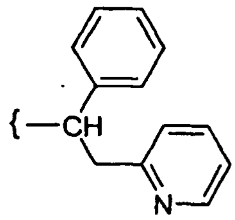 ,

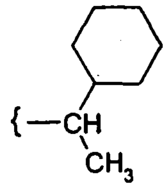 , 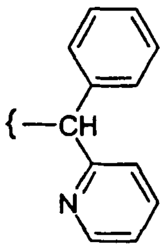 , 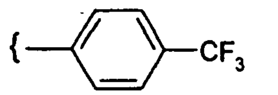 ,

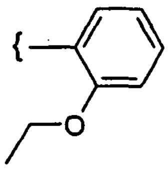 , 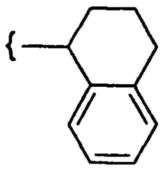 , 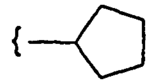 ,

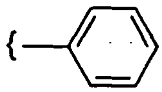 , 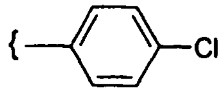 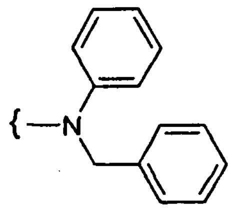

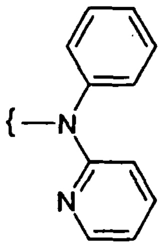 , oder 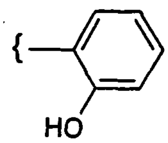 ,

bedeutet;

in Ij $R^1$, $R^{1'}$ H,

$R^2$ H, OA oder Hal,

$R^3$ A, Ar, Het, $NR^4R^5$ oder $CHR^4R^5$, wobei mindestens einer der Reste $R^4$ oder $R^5$ $(CH_2)_nAr$ oder $(CH_2)_nHet$ bedeutet,

$R^4$, $R^5$ jeweils unabhängig voneinander H, A, $(CH_2)_nAr$ oder $(CH_2)_nHet$,

X fehlt, $CH_2$ oder NH,

(fortgesetzt)

| | |
|---|---|
| Y | fehlt, |
| Ar | einen unsubstituierten oder ein-, zwei- oder dreifach durch OH, OA, Hal und/oder A substituierten gesättigten, ungesättigten oder aromatischen Carbocyclus mit 5-14 C-Atomen, |
| Het | einen einkernigen aromatischen Heterocyclus mit 1 bis 3N-, O und/oder S-Atom, |
| A | jeweils unabhängig voneinander unverzweigtes oder verzweigtes Alkyl mit 1-10 C-Atomen, worin 1-7 H-Atome durch F, Cl und/oder Br ersetzt sein können, Cycloalkyl mit 3-8 C-Atomen oder Cycloalkylalkylen mit 4-10 C-Atomen, |
| Hal | F, Cl, Br oder I, |
| n | 0, 1 oder 2 |
| | bedeuten; |
| in Ik $R^1$, $R^{1'}$ | H, |
| $R^2$ | H, OA oder Hal, |
| $R^3$ | A, Ar, Het, $NR^4R^5$ oder $CHR^4R^5$, wobei mindestens einer der Reste $R^4$ oder $R^5$ $(CH_2)_nAr$ oder $(CH_2)_nHet$ bedeutet, |
| $R^4$, $R^5$ | jeweils unabhängig voneinander H, A, $(CH_2)_nAr$ oder $(CH_2)_nHet$, |
| X | fehlt, $CH_2$ oder NH, |
| Y | fehlt, |
| Ar | unsubstituiertes oder ein-, zwei- oder dreifach durch OH, OA, Hal und/oder A substituiertes Phenyl, Naphthyl oder 1,2,3,4-Tetrahydronaphthalin, |
| Het | Pyridyl, Pyrimidinyl, Thienyl oder Furyl, |
| A | jeweils unabhängig voneinander unverzweigtes oder verzweigtes Alkyl mit 1-10 C-Atomen, worin 1-7 H-Atome durch F, Cl und/oder Br ersetzt sein können, Cycloalkyl mit 3-8 C-Atomen oder Cycloalkylalkylen mit 4-10 C-Atomen, |
| Hal | F, Cl, Br oder I, |
| n | 0, 1 oder 2 |
| | bedeuten; |

sowie ihre pharmazeutisch verwendbaren Salze, Solvate, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Verbindungen der Formel I können vorzugsweise erhalten werden, indem man Verbindungen der Formel II und mit Verbindungen der Formel III umsetzt.

Die Verbindungen der Formel II und der Formel III sind in der Regel bekannt. Sind sie neu, so können sie aber nach an sich bekannten Methoden hergestellt werden.

Die Umsetzung erfolgt in Gegenwart eines Carbodiimids, wie z.B. EDCI (N-Ethyl-N,N'-(dimethylaminopropyl)-carbodiimid) oder Dicyclohexylcarbodiimid, gegebenenfalls in Gegenwart einer organischen Base, wie z.B. N-Methylmorpholin und in einem inerten Lösungsmittel. Zweckmäßigerweise kann auch in situ ein aktivierter Ester gebildet werden, z. B. durch Zusatz von HOBt (Hydroxybenzotriazol) oder N-Hydroxysuccinimid.

In den Verbindungen der Formel III bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Derartige Reste zur Aktivierung der Carboxygruppe in typischen Acylierungsreaktionen sind in der Literatur (z.B. in den Standardwerken wie Houben-Weyl, Methoden der organischen Chemie, Georg-Thieme-Verlag, Stuttgart;) beschrieben.

Die Reaktionszeit liegt je nach den angewendeten Bedingungen zwischen einigen Minuten und 14 Tagen, die Reaktionstemperatur zwischen etwa -15° und 150°, normalerweise zwischen -5° und 90°, besonders bevorzugt zwischen 20° und 60°C.

Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether,

Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist THF, Dichlormethan und/oder DMF.

Verbindungen der Formel I können weiterhin erhalten werden, indem man Verbindungen der Formel II mit Verbindungen der Formel IV umsetzt.
Die Verbindungen der Formel IV sind in der Regel bekannt. Sind sie neu, so können sie aber nach an sich bekannten Methoden hergestellt werden. Die Umsetzung erfolgt in der Regel in einem inerten Lösungsmittel, in Gegenwart einer organischen Base wie Triethylamin, Dimethylanilin, Pyridin oder Chinolin. Die Reaktionszeit liegt je nach den angewendeten Bedingungen zwischen einigen Minuten und 14 Tagen, die Reaktionstemperatur zwischen etwa 0° und 150°, normalerweise zwischen 15° und 90°, besonders bevorzugt zwischen 15 und 30°C. Als inerte Lösungsmittel eignen sich die oben genannten.

Die Spaltung eines Ethers erfolgt unter Methoden, wie sie dem Fachmann bekannt sind.
Eine Standardmethode zur Etherspaltung, z.B. eines Methylethers, ist die Verwendung von Bortribromid.
Hydrogenolytisch entfernbare Gruppen, z.B. die Spaltung eines Benzylethers, können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.

Ester können z.B. mit Essigsäure oder mit NaOH oder KOH in Wasser, Wasser-THF oder Wasser-Dioxan bei Temperaturen zwischen 0 und 100° verseift werden.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer $NH_2$-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-Iodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).
Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°.
Die Tritylgruppe wird zum Schutz der Aminosäuren Histidin, Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt. Die Pbf (Pentamethylbenzofuranyl)-gruppe wird zum Schutz von Arg eingesetzt. Die Abspaltung erfolgt z.B. mit TFA in Dichlormethan.
Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.
Verbindungen der Formel I, worin X $NR^6$ oder O bedeutet, können vorzugsweise erhalten werden, indem man eine Verbindung der Formel V mit einem Kupplungsreagenz ausgewählt aus der Gruppe

a) Isoproylidenchloroformiat,
b) p-Nitrophenylchloroformiat,
c) Diphosgen,
d) Triphosgen,

und einer Verbindung der Formel VI umsetzt. Die Umsetzung erfogt vorzugsweise als Eintopfreaktion.
Die Umsetzung erfolgt in der Regel in einem inerten Lösungsmittel.
Die Reaktionszeit liegt je nach den angewendeten Bedingungen zwischen einigen Minuten und 14 Tagen, die Reaktionstemperatur zwischen etwa -15° und 150°, normalerweise zwischen -5° und 90°, besonders bevorzugt zwischen 20° und 60°C.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan, Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Besonders bevorzugt ist THF, Dichlormethan, Pyridin und/oder DMF.
Die Umsetzung erfolgt in der Regel in Gegenwart eines säurebindenden Mittels vorzugsweise einer organischen Base wie DIPEA, Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Anschließend erfolgt die Abspaltung der Schutzgruppe, wie oben angegeben.

Pharmazeutische Salze und andere Formen

Die genannten erfindungsgemäßen Verbindungen lassen sich in ihrer endgültigen Nichtsalzform verwenden.

Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie ($C_1$-$C_4$) Alkylhalogeniden, z.B. Methyl-, Ethyl-, Isopropyl- und tert.-Butylchlorid, -bromid und -iodid; Di($C_1$-$C_4$) Alkylsulfaten, z.B. Dimethyl-, Diethyl- und Diamylsulfat; ($C_{10}$-$C_{18}$)Alkylhalogeniden, z.B. Decyl-, Dodecyl-, Lauryl-, Myristyl- und Stearylchlorid, -bromid und -iodid; sowie Aryl-($C_1$-$C_4$)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz

auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels Iontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendem mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältem, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per* se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von

(a) einer wirksamen Menge an einer Verbindung der Formel I und/oder ihrer pharmazeutisch verwendbaren Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

VERWENDUNG

Die vorliegenden Verbindungen eignen sich als pharmazeutische Wirkstoffe für Säugetiere, insbesondere für den Menschen, bei der Behandlung und Bekämpfung von Krebserkrankungen.

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Himkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.

Ebenfalls umfasst ist die Verwendung der Verbindungen der Formel I und/oder ihre physiologisch unbedenklichen Salze und Solvate zur Herstellung eines Arzneimittels zur Behandlung und/oder Bekämpfung einer durch Tumore

bedingten Krankheit bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.

Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Gegenstand der Erfindung ist weiterhin die Verwendung der erfindungsgemäßen Verbindungen zur Behandlung von Knochen-Pathologien, wobei die Knochenpathologie aus der Gruppe Osteosarkom, Osteoarthritis und Rachitis stammt.

Die Verbindungen der Formel I können auch gemeinsam mit anderen gut bekannten Therapeutika, die aufgrund ihrer jeweiligen Eignung für das behandelte Leiden ausgewählt werden, verabreicht werden.
Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. "Östrogenrezeptormodulatoren" bezieht sich auf Verbindungen, die die Bindung von Östrogen an den Rezeptor stören oder diese hemmen, und zwar unabhängig davon, wie dies geschieht. Zu den Östrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1-piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5$\alpha$-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, $\alpha$-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumomekrosefaktoren, interkalierende Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyldaunorubicin (siehe WO 00/50032), was jedoch keine Einschränkung darstellen soll.
Zu den Mikrotubulin-Hemmem zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Hemmer sind zum Beispiel Topotecan, Hycaptamin, Irinotecan, Rubitecan, 6-Ethoxypropionyl-3',4'-O-exo-benzylidenchartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13 (9H,15H)-dion, Lurtotecan, 7-[2-(N-Isopropylamino)ethyl]-(20S)camptothecin, BNP1350, BNPI1100, BN80915,

BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4': 6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxämid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-mannoheptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diazatetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehydthiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

**Wirkungsnachweis von pharmakologischen Inhibitoren auf die Proliferation von Tumorzellen *in vitro***

## 1.0 Hintergrund

In der vorliegenden Versuchsbeschreibung wird die Hemmung der Tumorzellproliferation durch Wirkstoffe beschrieben.
Die Zellproliferation wird durch Messung der Propidium Jodidaufnahme geschätzt. Propidium Jodid ist in gelöstem, freien Zustand farblos und fluoresziert nicht. Es wird von gesunden Zellen vom Eintritt in die Zellen ausgeschlossen. Wenn aber Zellen durch Zugabe des Detergens NP40 abgestorben sind, werden Zellmembran und Kernmembran durchlässig. PI lagert sich an die DNA der Zellkerne an und fluoresziert in diesem Komplex. Diese Fluoreszenz wird im Fluorimeter gemessen.

## 2.0 Versuchsdurchführung

### 2.1 Zellkultur

käuflich erhältliche Colon-Carcinom-Zelllinien

#### 2.1.1 Ablösen adhärent wachsender Zellen (Passage)

#### 2.1.2 Bestimmung der Zellzahl und Vitalität

### 2.2 Aussaat der Zellen für den Versuch

Es werden 10.000 Zellen je Napf, in einem Volumen von 180µl, in einer 96-Lochplatte ausplatiert. Dabei werden die äußeren Reihen A1-12, 1A-H, 12A-H und H1-12 nur mit 200µl Medium oder Puffer gefüllt.

#### 2.2.1 Präinkubation der Zellen

Die Zellen werden 24 Stunden, bei 37° C und 10%iger $CO_2$-Begasung inkubiert.

### 2.3 Plattenschema und Zusatz von Substanzen:

Um Wirksubstanzen zu testen, müssen zunächst die entsprechenden Verdünnungen erstellt und den Näpfchen zugeordnet werden. Das als Beispiel hier angegebene Schema einer 1:3 Verdünnung kann je nach Wunsch entsprechend variiert werden. Das beschriebene Schema ist für 4 Testsubstanzen ausgelegt.

Die Anordnung ist nur ein Vorschlag. Je nach Fragestellung kann auch eine andere Aufteilung gewählt werden.

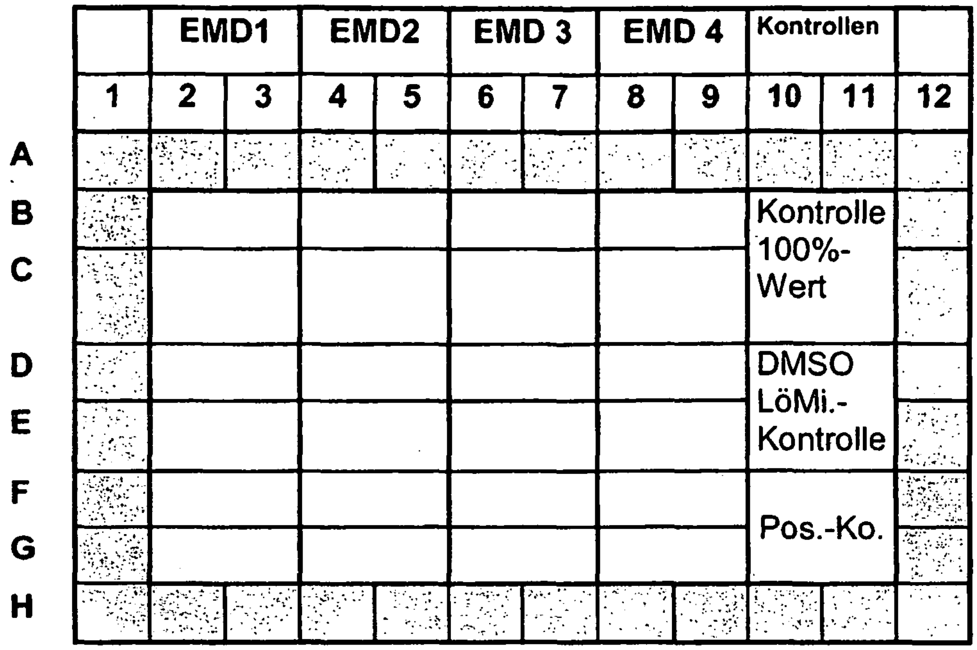


<u>100%-Werte, LöMi. - Kontrolle bzw. Positiv-Kontrolle (Spalte B-G, Näpfchen 10+11)</u>: In diese Löcher werden 20µl Medium, Lösungsmittelverdünnung bzw. die entsprechende Verdünnung der Positivkontrolle (IC50) pipettiert.

<u>Substanzgruppen (Spalte B-G, Näpfchen 2-9):</u> In diese Löcher werden 20µl Substanzverdünnung pipettiert. Substanzverdünnungsreihe in Extraplatte (Beispiel für 1:3 Verdünnung):

In den Näpfen der Reihe B der Extraplatte wird in den Spalten 1, 2, 3, 4 jeweils 147µl Medium vorgelegt und 3µl der Testsubstanz zugegeben (=300µM). In den Reihen C - G, Spalten 1 - 4 werden 100µl Medium vorgelegt. Nun werden 50µl mit einer Mehrkanalpipette in Reihe C übertragen, 8x gemischt und dann werden 50µl aus dieser Reihe in Reihe D übertragen. So wird sinngemäß fortgefahren, bis die Reihe G erreicht ist, bei der nach dem Mischen 50µl der Verdünnung entnommen und verworfen werden. Jetzt befinden sich 100µl jeder gewünschten Verdünnung in der Extraplatte und es müssen jetzt nur noch 20µl, direkt in die Testplatte übertragen werden.

**EMDs:** 15 mM DMSO

**Gesamtvolumen/Well [µl]:** 200,0

| | | | | | | Bei Plattenverdünnung | | | | |
|---|---|---|---|---|---|---|---|---|---|---|
| | | | Vol. Verd. [µl] 20 | | | | Mln. Vol. / Well | | | |
| **Testkonzentration / Well** | | | **Konzentrat** | | | Verd. Faktor 1: | Stckl. | | 100 [µl] | 150µl von 1) |
| 1) | 30,0000 | µM | 300,0000 | µM | → | 50 | 3,0µl | + | 147 | µl Medium |
| 2) | 10,0000 | µM | 100,0000 | µM | → | 3 | 50µl von 1) | + | 100 | µl Medium |
| 3) | 3,3333 | µM | 33,3333 | µM | → | 3 | 50µl von 2) | + | 100 | µl Medium |

(fortgesetzt)

| | | | | | | Bei Plattenverdünnung | | | | |
|---|---|---|---|---|---|---|---|---|---|---|
| | **Testkonzentration / Well** | | Vol. Verd. [µl] 20 **Konzentrat** | | | Verd. Faktor 1: | Stckl. | | Mln. Vol. / Well 100 [µl] | 150µl von 1) |
| 4) | 1,1111 | µM | 11,1111 | µM | → | 3 | 50µl von 3) | + | 100 | µl Medium |
| 5) | 0,3704 | µM | 3,7037 | µM | → | 3 | 50µl von 4) | + | 100 | µl Medium |
| 6) | 0,1235 | µM | 1,2346 | µM | → | 3 | 50µl von 5) | + | 100 | µl Medium |
| 7) | 0 | µM | 0 | µM | | | | | | |

**2.4 Inkubation** der **Zellen**

Die Platte wird bei 37° C und 10%iger CO2-Begasung 3 Tage inkubiert. Die in dieser Zeit sich vermehrenden Zellen, werden mittels Propidium Jodid ermittelt.

**2.5 Messung der Zellproliferation mittels Propidium Jodid**

**2.5.1 Zusatz von PI und NP40**

Pro Well werden 20µl PI Stammlösung und 10 µl NP40 Stammlösung benötigt. Da jede Platte 72 Näpfchen hat, die mit PI und mit NP40 beschickt werden, benötigt man 1440µl der PI-Stocklösung und 720µl der NP40-Stocklösung pro Platte. Unter Einbezug von Pipettierverlusten nimmt man also 1,8ml PI Stocklösung und vermischt diese unmittelbar vor dem Einpipettieren mit 0,9ml der NP40-Stocklösung. In jedes der Näpfchen der Platte, die jeweils 200µl Medium enthalten, werden nun 30µl der PI/NP40-Mischung pipettiert. Die Platte wird anschließend für 2 Stunden in den Brutschrank gestellt.

**2.5.2 Messung und Berechnung**

Die Platte(n) wird in einem Floureszenzfotometer bei einer Wellenlänge Ex. 520nm und Em von 620nm gemessen. Allen Werten wird der Hintergrund (= Medium + PI) abgezogen, anschließend gemittelt (Zweierwerte) und in % der Kontrolle ausgedrückt bzw. berechnet.

$$\text{Rechnung:}\quad \frac{100 * (\text{Mittelwert der Einzelwerte - Mittelwert Hintergrund})}{(\text{Mittelwert der 100\% Einzelwerte - Mittelwert Hintergrund})}$$

Jetzt kann man mit einem Statistikprogramm wie z.B. RS1, eine $IC_{50}$ (Halbmaximale Wirkung) berechnen.

$IC_{50}$-Daten einiger erfindungsgemäßer Verbindungen sind in Tabelle 1 angegeben.

**3.0 Materialien, Reagentien und Lösungen**

| **3.1 Materialien** | **Firma / Kat.Nr.** |
|---|---|
| Fluoroskan *Ascent* | Labsystems S.-Nr.: 374 009-751 |
| 8-Kanal-Pipette (z. B. Finnpipette, 5 - 50 µl) | Kühn u. Bayer 4142407 |
| 8-Kanal-Pipette (z. B. Finnpipette, 50 - 300 µl) | Kühn u. Bayer 4142417 |
| 12-Kanal-Pipette (z. B. Finnpipette, 5 - 50 µl) | Kühn u. Bayer 4172307 |
| 12-Kanal-Pipette (z. B. Finnpipette, 50 - 300 µl) | Kühn u. Bayer 4172317 |

(fortgesetzt)

| 3.1 Materialien | Firma / Kat.Nr. |
|---|---|
| Multipette 4780 | Eppendorf 4780 |
| Varipette 200 -1000 µl | Eppendorf 4810 |
| Varipette 10 -100 µl | Eppendorf 4810 |
| Varipette 0,5 -10 µl | Eppendorf 4810 |
| sterile Spitzen, Standardtips 10 µl | Eppendorf 22195 |
| sterile Spitzen, Standardtips 1000 µl | Eppendorf 35443 |
| sterile Spitzen, BR-38 100 µl | Bio-Rad 223-9038 |
| Polypropylenröhrchen, 50 ml, steril | Falcon 2070 |
| Polypropylenröhrchen, 6 ml, steril | Falcon 2063 |
| Polypropylenröhrchen, 14 ml, steril | Falcon 2059 |
| 96-Näpfchen Mikrotiterplatte für Gewebekultur, steril | Nunc 167008 |
| Reagent Reservoir, steril | Costar 4870 |
| Reaktionsgefäße, 1,5 ml Polypropylen | Eppendorf 3810 |
| Sterilfilter (Bottle Top Filter) 0,22 µm | Falcon 7105 |
| **3.2 Reagentien und Stammlösungen** | **Firma / Kat.Nr.** |
| *Kulturmedium:* | |
| MEM ALPHA MEDIUM | Gibco |
| Lagerung bei +4°C | 22571-020 |
| Fetales Kälberserum (FCS), steril | Gibco |
| portioniert: 50 ml in 50 ml Polypropylenröhrchen | 011-06290 M |
| Lagerung bei -20°C | |
| Penicillin / Streptomycin | Gibco |
| Lagerung bei -20°C | 15140-114 |
| L-Glutamine (200 mM) | Gibco |
| Lagerung bei -20°C | 25030-024 |
| Nonidet® P40 | |
| [Nonylphenyl-polyethyleneglycol] | Bio Chemika 74385 |
| Propidium Iodide | Sigma P4170 |
| DMSO (Dimethylsulfoxid) für die Spektroskopie | Merck 2950 |

**3.3 Ansetzen von Lösungen**

**3.3.1 Kulturmedium:**

500 ml (1 Flasche) alpha-MEM
+ 50 ml FCS = 10 %
+ 5 ml Penicillin / Streptomycin
+ 5 ml Glutamin = 2 mM
Lagerung bei +4°C

**3.3.2 Prüfsubstanzen:**

Gewünschte Stammlösung bei 15 µM in DMSO ansetzen. Durch Verdünnen in Kultürmedium auf das Zehnfache der Konzentration bringen, die in der Zellkultur getestet werden soll (s.o.).

**3.3.3 Propidium Iodid-Stammlösung**

20mg PI in 20ml PBS lösen und Röhrchen unter Lichtschutz (in Alufolie einwickeln) bei 4°C aufbewahren.

**3.3.4 NP40 Stammlösung**

11 %ig gelöst in Wasser und bei 4°C aufbewahren

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-Ionisation) $M^+$ |
| | FAB (Fast Atom Bombardment) $(M+H)^+$ |
| | ESI (Electrospray Ionization) $(M+H)^+$ |

APCI-MS (atmospheric pressure chemical ionization - mass spectrometry) $(M+H)^+$.

HPLC-Gradientensystem

Säule:

ChromolithPerformance RP-18e (Merck KGaA, Cat. 1.02129.0001)

Eluenten:

Eluent A: 0.1 M wässr. NaH2PO4
Eluent B: Acetonitril + 10 % Wasser
Flußrate: 4 ml/min

Gradient:

0 min 1 % B
1 min 1 % B
7 min 99 % B
8 min 99 % B

**Beispiel 1**

Die Herstellung von *N*-(5-Chlor-8-hydroxy-chinolin-7-yl)-3,3-diphenylpropionamid ("A1") erfolgt analog nachstehendem Schema

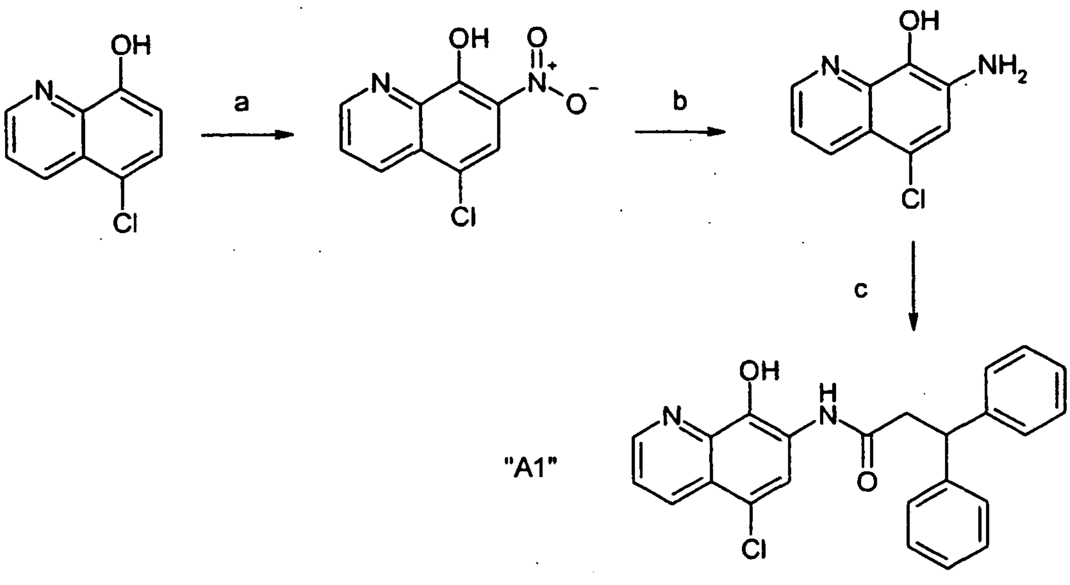


"A1"

a) 10 g 5-Chlor-chinolin-8-ol werden in 56 ml konz. $H_2SO_4$ gelöst, unter Kühlen u. Rühren bei max. 0 °C ein Gemisch aus 100 % 2,8 ml $HNO_3$ + 0,5 ml $H_2O$ zugetropft und anschließend 2 h ohne Kühlung nachgerührt.
Aufarbeitung: Es wird in 300 ml Eis / $H_2O$ eingerührt, die entstandene Fällung abgetrennt, mehrmals mit $H_2O$ gewaschen, in wenig kaltem MeOH angerührt, abgesaugt und mit Ether gewaschen.
Man erhält 10,2 g 5-Chlor-7-nitro-chinolin-8-ol (81 %), F. 201 - 202°; HPLC: RT 5,36 Min.

b) In einem $N_2$-gespülten 500 ml Dreihalskolben werden 10,135 g 5-Chlor-7-nitro-chinolni-8-ol in einem Gemisch aus 150 ml MeOH und 150 ml $H_2O$ suspendiert, unter Rühren portionsweise 40,54 g Natriumdithionit zugegeben und 16 h bei RT nachgerührt, wobei sich die Suspension von intensiv orange nach blassgelb färbt.
Es wird abgesaugt, mit reichlich $H_2O$ gewaschen und bei 70 °C getrocknet. Ausbeute: 8,16 g (93%) 5-Chlor-7-amino-chinolin-8-ol, F. 165-166°; HPLC:
RT 4,77 Min.

c) 1,131 g 3,3-Diphenylpropionsäure wird in einem 100 ml Einhalskolben in 10 ml abs.THF vorgelegt, 0,892 g 1,1'-Carbonyldiimidazol zugegeben, 2 h bei RT gerührt, dann 0,973 g 5-Chlor-7-amino-chinolin-8-ol zugegeben und 20 h unter Rückfluss gerührt.
Es wird mit 50 ml $H_2O$ verdünnt, 10 min kräftig gerührt, die entstandene Fällung abgesaugt, mit wenig MeOH gewaschen und über 50 g Kieselgel chromatographiert.
Man erhält 1,155 g (57%) "A1", F. 206-207°;
$^1H$ NMR (250 MHz, DMSO-$d_6$) $\delta$ 10.451 (SB, 1H), 9.710 (S, 1H). 8.930 (D, 1H), 8.426 (D, 1H), 8.243 (S, 1H), 7.631 (Q, 1H), 7.375 (M, 4H), 7.292 (M, 4H), 7.170 (T, 2H), 4.604 (T, 1H), 3.335 (D, 2H).

Analog erhält man die nachstehenden Verbindungen

| Verbindung Nr. | Struktur / Name | analytical data |
|---|---|---|
| "A2" | 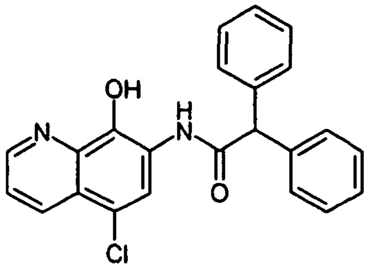 *N*-(5-Chlor-8-hydroxy-chinolin-7-yl)-2,2-diphenylacetamid | F. 217-218° $^1$H NMR (250 MHz, DMSO-$d_6$) $\delta$ 10.62 (S, 1H), 10.045 (S, 1H), 8.934 (DD, 1H), 8.452 (M, 2H), 7.646 (Q, 1H), 7.331 (M, 10H), 5.647 (S, 1H) |
| "A3" | 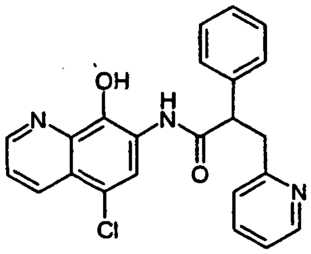 *N*-(5-Chlor-8-hydroxy-chinolin-7-yl)-2-phenyl-3-pyridin-2-yl-propionamid | F. 219-220° $^1$H NMR (250 MHz, DMSO-$d_6$) $\delta$ 10.505 (SB, 1H), 9.796 (S, 1H), 8.911 (D, 1H), 8.430 (M, 2H), 8.351 (M, 2H), 7.631 (M, 2H), 7.491 (D, 2H), 7.331 (T, 2H), 7.255 (M, 2H), 4.526 (T, 1H), 3.413 (DD, 1H), 3.053 (DD, 1H) |
| "A4" | 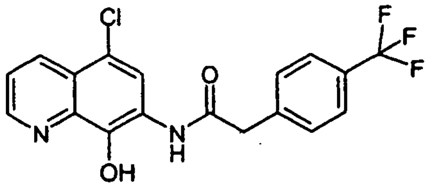 *N*-(5-Chlor-8-hydroxy-chinolin-7-yl)-2-(4-trifluormethyl-phenyl)-acetamid | |
| "A5" | 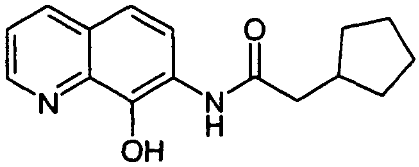 2-Cyclopentyl-N-(8-hydroxy-chinolin-7-yl)-acetamid | |
| "A6" | 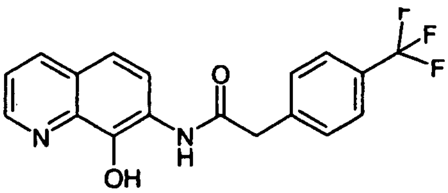 *N*-(8-Hydroxy-chinolin-7-yl)-2-(4-trifluormethylphenyl)-acetamid | |
| "A7" | 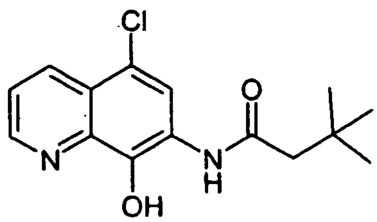 *N*-(5-Chlor-8-hydroxy-chinolin-7-yl)-3,3-dimethylbutyramid | |

(fortgesetzt)

| Verbindung Nr. | Struktur / Name | analytical data |
|---|---|---|
| "A8" | 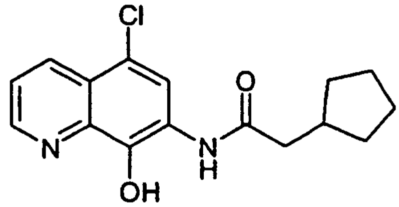 *N*-(5-Chlor-8-hydroxy-chinolin-7-yl)-2-cyclopentyl-acetamid | |
| "A9" | 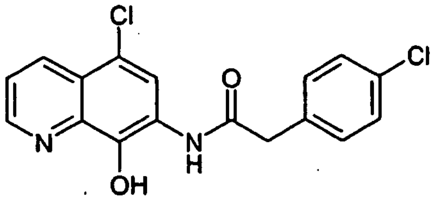 *N*-(5-Chlor-8-hydroxy-chinolin-7-yl)-2-(4-chlorphenyl)-acetamid | |
| "A10" | 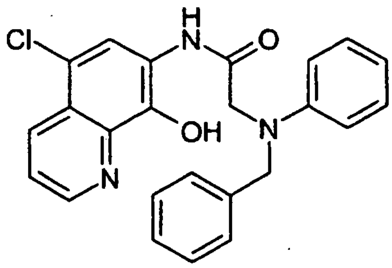 2-(Benzyl-phenyl-amino)-*N*-(5-chlor-8-hydroxy-chinolin-7-yl)-acetamid | |
| "A11" | 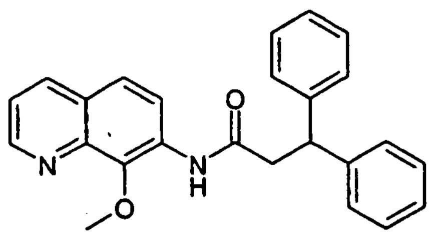 *N*-(8-Methoxy-chinolin-7-yl)-3,3-diphenyl-propionamid | |
| "A12" | 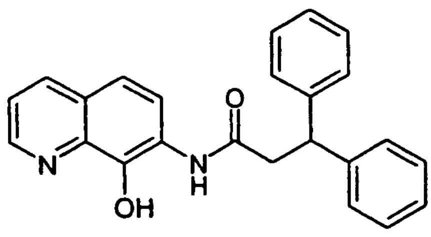 *N*-(8-Hydroxy-chinolin-7-yl)-3,3-diphenylpropionamid [aus "A11" durch Etherspaltung mit $BI_3$] | |

(fortgesetzt)

| Verbindung Nr. | Struktur / Name | analytical data |
| --- | --- | --- |
| "A13" | 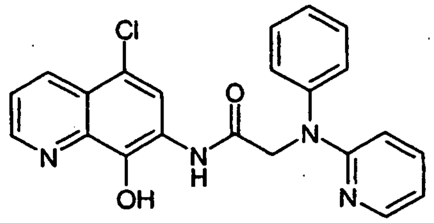 *N*-(5-Chlor-8-hydroxy-chinolin-7-yl)-2-(phenyl-pyridin-2-yl-amino)-acetamid | |

Beispiel 2

Die Herstellung von 1-(5-Chlor-8-hydroxy-chinolin-7-yl)-3-(phenyl-pyridin-2-yl-methyl)-harnstoff ("A14") erfolgt analog nachstehendem Schema

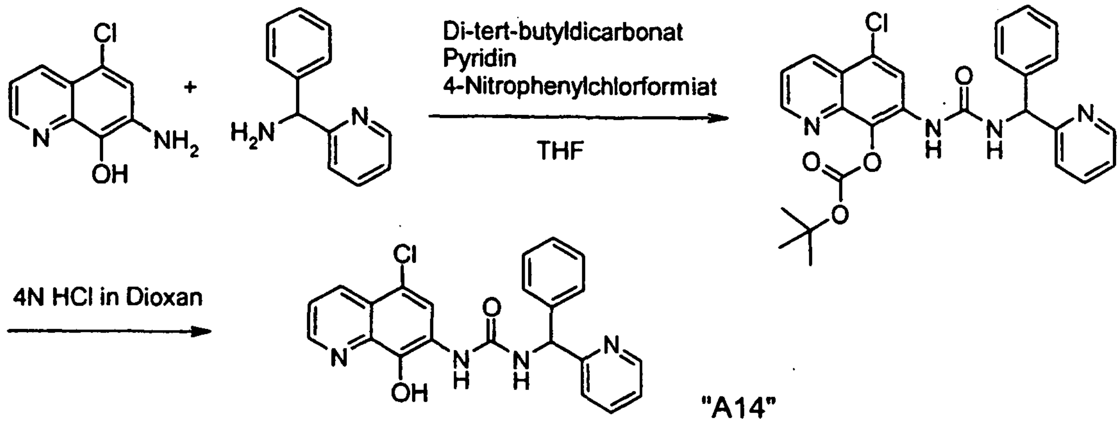


"A14"

Dihydrochlorid

2.1 a) Eine Lösung von 389 mg (2.00 mmol) 7-Amino-5-chlor-chinolin-8-ol in 5 ml THF wird mit 480 mg (2.20 mmol) Di-tert-butyldicarbonat und 179 μl Pyridin versetzt und 18 Stunden bei Raumtemperatur gerührt. Dann werden 403 mg (2.00 mmol) 4-Nitrophenylchlorformiat zugegeben und 1 Stunde bei Raumtemperatur gerührt.

2.1 b) Dann wird 368 mg (2.00 mmol) C-Phenyl-C-pyridin-2-yl-methylamin zugegeben und 5 Tage bei Raumtemperatur gerührt. Das Lösungsmittel wird abdestilliert, der Rückstand in Dichlormethan aufgenommen und mit 0.1 N HCl und 0.1 N NaOH gewaschen. Die organische Phase wird über Natriumsulfat getrocknet, eingedampft und der Rückstand an einer Kieselgelsäule chromatographiert: Carbonsäure-tert-butylester-5-chlor-7-[3-(phenylpyridin-2-yl-methyl)-ureido]-chinolin-8-yl-ester als braunes hochviskoses Öl; ESI 505.

2.2 550 mg (0.523 mmol) Carbonsäure-tert-butylester-5-chlor-7-[3-(phenylpyridin-2-yl-methyl)-ureido]-chinolin-8-yl-ester werden in 10 ml einer 4N Lösung von Chlorwasserstoff in Dioxan gelöst und 18 Stunden bei Raumtemperatur gerührt. Das Lösungsmittel wird abdestilliert und der Rückstand chromatographiert: 1-(5-Chlor-8-hydroxychinolin-7-yl)-3-(phehyl-pyridin-2-yl-methyl)-harnstoff Dihydrochlorid als braune Kristalle; ESI 405.

$^1$H-NMR ($d_6$-DMSO): δ = 6.02 (d, J = 6.5 Hz, 1H), 7.30 (t, J = 7.3 Hz, 1H), 7.38 (t, J = 7.5 Hz, 2H), 7.49 (d, J = 7.5 Hz, 2H), 7.56 (m, 1H), 7.60 (dd, $J_1$ = 16 Hz, $J_2$ = 8.5 Hz, 1H), 7.81 (d, J = 7.5 Hz, 1H), 8.12 (t, J = 6.5 Hz, 1H), 8.44 (d, J = 6.5 Hz, 1H), 8.47 (d, J = 8.5 Hz, 1H), 8.63 (s, 1H), 8.68 (d, J = 4.5 Hz), 8.92 (d, J = 3 Hz, 1H), 9.08 (s, 1H), 10.6 (bs, 1H).

Analog erhätt man die nachstehenden Verbindungen

| Verbindung Nr. | Struktur / Name | |
|---|---|---|
| "A15" | 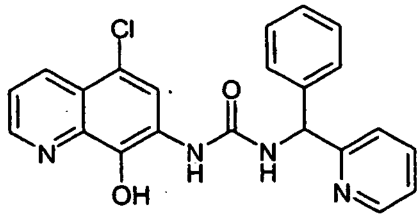<br>1-(5-Chlor-8-hydroxy-chinolin-7-yl)-3-(phenyl-pyridin-2-yl-methyl)-harnstoff | |
| "A16" | 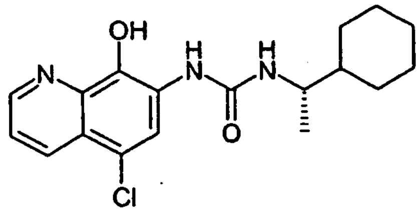<br>1-(5-Chlor-8-hydroxy-chinolin-7-yl)-3-((S)-1-cyclohexyl-ethyl)-harnstoff | |
| "A17" | 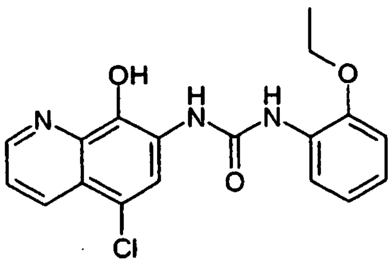<br>1-(5-Chlor-8-hydroxy-chinolin-7-yl)-3-(2-ethoxy-phenyl)-harnstoff | |
| "A18" | 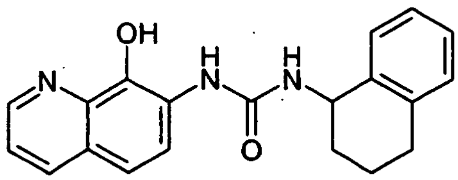<br>1-(8-Hydroxy-chinolin-7-yl)-3-(1,2,3,4-tetrahydro-naphthalin-1-yl)-harnstoff | |
| "A19" | 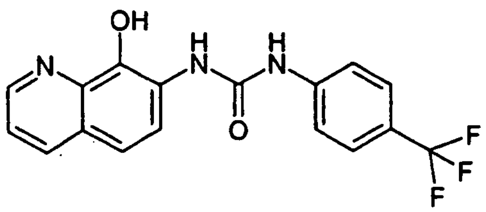<br>1-(8-Hydroxy-chinolin-7-yl)-3-(4-trifluormethyl-phenyl)-harnstoff | |
| "A20" | 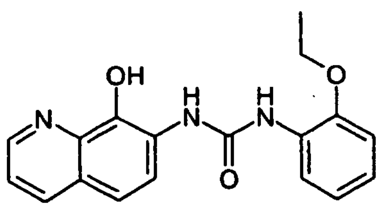<br>1-(2-Ethoxy-phenyl)-3-(8-hydroxy-chinolin-7-yl)-harnstoff | |

(fortgesetzt)

| Verbindung Nr. | Struktur / Name | |
|---|---|---|
| "A21" | 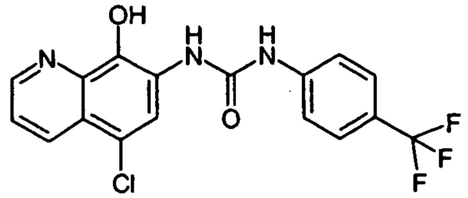 1-(5-Chlor-8-hydroxy-chinolin-7-yl)-3-(4-trifluormethyl-phenyl)-harnstoff | |
| "A22" | 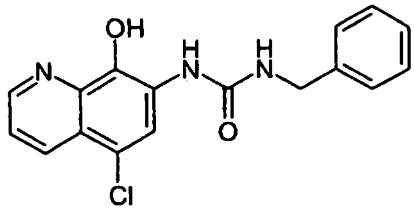 1-Benzyl-3-(5-chlor-8-hydroxy-chinolin-7-yl)-harnstoff | |

Beispiel 3

Die Herstellung von 1-(2-Hydroxy-phenyl)-3-(8-hydroxy-chinolin-7-yl)-hamstoff ("A24") erfolgt analog nachstehendem Schema

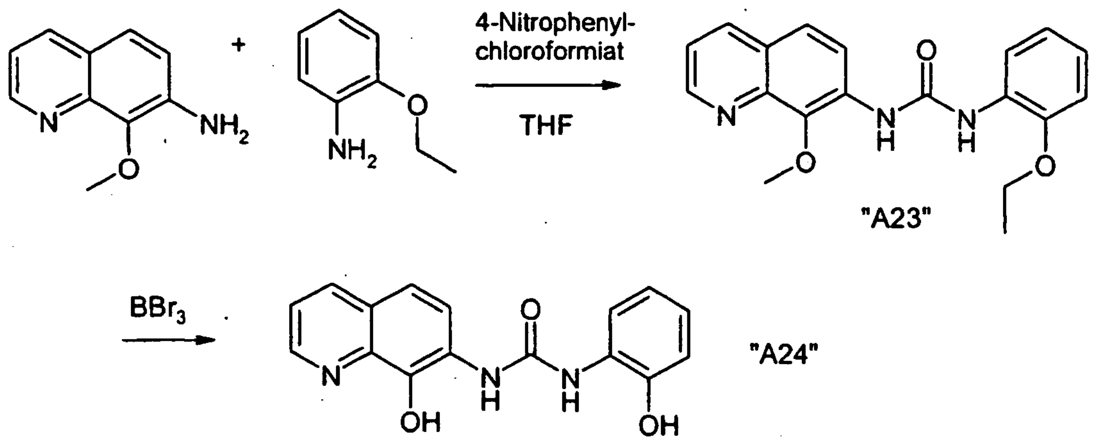


Beispiel 4

Die Herstellung von 1-(2-Trifluormethyl-phenyl)-3-(8-hydroxy-chinolin-7-yl)-hamstoff ("A26") erfolgt analog nachstehendem Schema

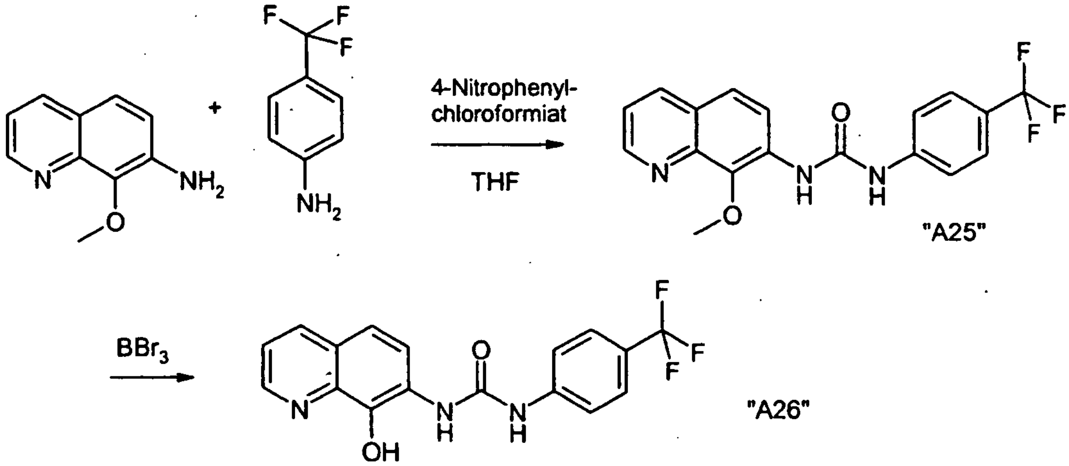


"A25"

"A26"

Tabelle 1

| Inhibierung auf die Proliferation von Tumorzellen $IC_{50}$ [mol/l] | |
|---|---|
| Verbindung Nr. | $IC_{50}$ |
| "A1" | 9.00E-08 |
| "A2" | 2.10E-06 |
| "A3" | 1.10E-06 |
| "A14" | 9.00E-08 |
| "A19" | 1.50E-06 |
| "A20" | 2.10E-07 |

Die nachfolgenden Beispiele betreffen Arzneimittel:

**Beispiel A: Injektionsgläser**

Eine Lösung von 100 g eines Wirkstoffes der Formel I und 5 g Dinatriumhydrogenphosphat wird in 3 l zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

**Beispiel B: Suppositorien**

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

**Beispiel C: Lösung**

Man bereitet eine Lösung aus 1 g eines Wirkstoffes der Formel I, 9,38 g $NaH_2PO_4 \cdot 2\ H_2O$, 28,48 g $Na_2HPO_4 \cdot 12\ H_2O$ und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

**Beispiel D: Salbe**

Man mischt 500 mg eines Wirkstoffes der Formel I mit 99,5 g Vaseline unter aseptischen Bedingungen.

**Beispiel E: Tabletten**

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

**Beispiel F: Dragees**

Analog Beispiel E werden Tabletten gepreßt, die anschließend in üblicher Weise mit einem Überzug aus Saccharose, Kartoffelstärke, Talk, Tragant und Farbstoff überzogen werden.

**Beispiel G: Kapseln**

2 kg Wirkstoff der Formel I werden in üblicher Weise in Hartgelatinekapseln gefüllt, so daß jede Kapsel 20 mg des Wirkstoffs enthält.

**Beispiel H: Ampullen**

Eine Lösung von 1 kg Wirkstoff der Formel I in 60 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I

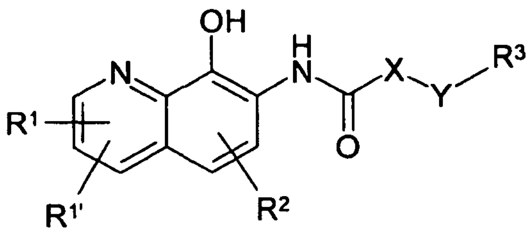

I

worin

$R^1$, $R^{1'}$, $R^2$ jeweils unabhängig voneinander H, OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA oder CN,
$R^3$ A, Ar, Het, $NR^4R^5$ oder $CHR^4R^5$,
wobei mindestens einer der Reste

$R^4$ oder $R^5$ $(CH_2)_nAr$ oder $(CH_2)_nHet$ bedeutet,

$R^4$, $R^5$ jeweils unabhängig voneinander H, A, $(CH_2)_nAr$ oder $(CH_2)_nHet$,
X fehlt, $CH_2$, $NR^6$ oder O,
Y fehlt oder Alkylen mit 1-3 C-Atomen,
$R^6$ H oder A,
A, A' jeweils unabhängig voneinander unverzweigtes oder verzweigtes Alkyl mit 1-10 C-Atomen,

worin 1-7 H-Atome durch F, Cl und/oder Br ersetzt

sein können,
Cycloalkyl mit 3-8 C-Atomen oder
Cycloalkylalkylen mit 4-10 C-Atomen,
Hal F, Cl, Br oder I,
Ar einen unsubstituierten oder ein-, zwei- oder dreifach durch OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA, COA, CHO oder CN substituierten gesättigten, ungesättigten oder aromatischen Carbocyclus mit 5-14 C-Atomen,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$,

COOH, COOA, CHO, COA, CN, =S, =NH, =NA und/oder =O (Carbonylsauerstoff) substituiert sein kann,
n 0, 1 oder 2,

bedeuten,
sowie ihre pharmazeutisch verwendbaren Solvate, Salze, Tautomere und Stereoisomere, einschließlich deren Mischungen in allen Verhältnissen
wobei die nachfolgenden Verbindungen ausgenommen sind

N-(8-Hydroxy-7-chinolyl)-benzamid,
N-(8-Hydroxy-7-chinolyl)-octanamid,
2-(Acetylamino)-N-(8-hydroxy-7-chinolyl)-acetamid,
N-(8-Hydroxy-7-chinolyl)-N'-phenyl-harnstoff,
N-(8-Hydroxy-7-chinolyl)-N'-octyl-harnstoff,
N-(8-Hydroxy-7-chinolyl)-N'-(phenylmethyl)-harnstoff,
N-(8-Hydroxy-7-chinolyl)-acetamid,
N-(5-Chlor-8-hydroxy-7-chinolyl)-2-methyl-propanamid,
4-[[[(5-Chlor-8-hydroxy-7-chinolyl)amino]carbonyl]amino]-N,N-dioctylbenzolsulfonamid,
7-Acetamido-8-hydroxy-5-chinolin-carbonsäure,
7-Acetamido-5-methyl-8-chinolinol.

**2.** Verbindungen nach Anspruch 1, worin

$R^1$, $R^{1'}$ H bedeuten,

sowie ihre pharmazeutisch verwendbaren Solvate, Salze, Tautomere und Stereoisomere, einschließlich deren Mischungen in allen Verhältnissen.

**3.** Verbindungen nach Anspruch 1 oder 2, worin

$R^2$ H, OA oder Hal bedeutet,

sowie ihre pharmazeutisch verwendbaren Solvate, Salze, Tautomere und Stereoisomere, einschließlich deren Mischungen in allen Verhältnissen.

**4.** Verbindungen nach Anspruch 1, 2 oder 3, worin

Ar einen unsubstituierten oder ein-, zwei- oder dreifach durch OH, OA, Hal und/oder A substituierten gesättigten, ungesättigten oder aromatischen Carbocyclus mit 5-14 C-Atomen,

bedeutet,
sowie ihre pharmazeutisch verwendbaren Solvate, Salze, Tautomere und Stereoisomere, einschließlich deren Mischungen in allen Verhältnissen.

**5.** Verbindungen nach einem oder mehreren der Ansprüche 1-4, worin

Het einen einkernigen aromatischen Heterocyclus mit 1 bis 3 N-, O und/oder S-Atomen

bedeutet,
sowie ihre pharmazeutisch verwendbaren Solvate, Salze, Tautomere und Stereoisomere, einschließlich deren Mischungen in allen Verhältnissen.

**6.** Verbindungen nach einem oder mehreren der Ansprüche 1-5, worin

X fehlt, $CH_2$ oder NH bedeutet,

sowie ihre pharmazeutisch verwendbaren Solvate, Salze, Tautomere und Stereoisomere, einschließlich deren Mischungen in allen Verhältnissen.

**7.** Verbindungen nach einem oder mehreren der Ansprüche 1-6, worin

Y fehlt bedeutet,

sowie ihre pharmazeutisch verwendbaren Solvate, Salze, Tautomere und Stereoisomere, einschließlich deren Mischungen in allen Verhältnissen.

**8.** Verbindungen nach einem oder mehreren der Ansprüche 1-7, worin

Ar unsubstituiertes oder ein-, zwei- oder dreifach durch OH, OA, Hal und/oder A substituiertes Phenyl, Naphthyl oder 1,2,3,4-Tetrahydronaphthalin

bedeutet,
sowie ihre pharmazeutisch verwendbaren Solvate, Salze, Tautomere und Stereoisomere, einschließlich deren Mischungen in allen Verhältnissen.

**9.** Verbindungen nach einem oder mehreren der Ansprüche 1-8, worin

Het Pyridyl, Pyrimidinyl, Thienyl oder Furyl,

bedeutet,
sowie ihre pharmazeutisch verwendbaren Solvate, Salze, Tautomere und Stereoisomere, einschließlich deren Mischungen in allen Verhältnissen.

**10.** Verbindungen nach einem oder mehreren der Ansprüche 1-9, worin $R^3$

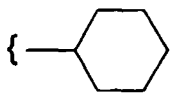 , 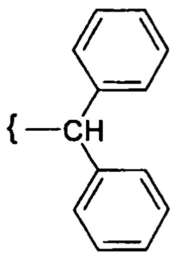 , 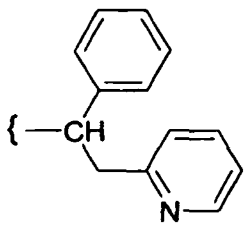 ,

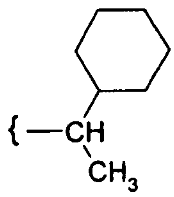 , 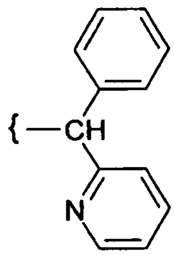 , 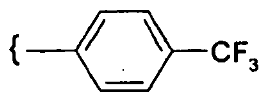 ,

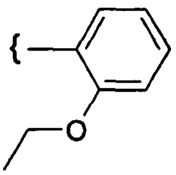,
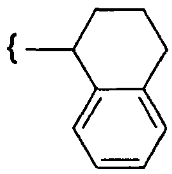,
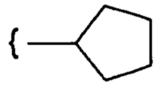,

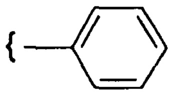,
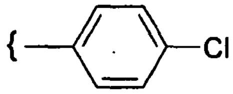
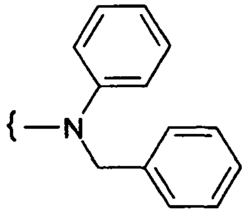,

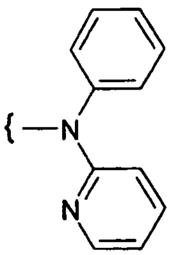, oder
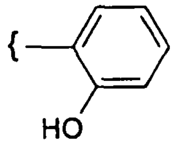,

bedeutet,
sowie ihre pharmazeutisch verwendbaren Solvate, Salze, Tautomere und Stereoisomere, einschließlich deren Mischungen in allen Verhältnissen.

**11.** Verbindungen nach einem oder mehreren der Ansprüche 1-10, worin

$R^1$, $R^{1'}$ H,
$R^2$ H, OA oder Hal,
$R^3$ A, Ar, Het, $NR^4R^5$ oder $CHR^4R^5$, wobei mindestens einer der Reste

$R^4$ oder $R^5$ $(CH_2)_nAr$ oder $(CH_2)_nHet$ bedeutet,

$R^4$, $R^5$ jeweils unabhängig voneinander H, A, $(CH_2)_nAr$ oder $(CH_2)_nHet$,
X fehlt, $CH_2$ oder NH,
Y fehlt,
Ar einen unsubstituierten oder ein-, zwei- oder dreifach durch OH, OA, Hal und/oder A substituierten gesättigten, ungesättigten oder aromatischen Carbocyclus mit 5-14 C-Atomen,
Het einen einkernigen aromatischen Heterocyclus mit 1 bis 3 N-, O und/oder S-Atom,
A jeweils unabhängig voneinander unverzweigtes oder verzweigtes Alkyl mit 1-10 C-Atomen,

worin 1-7 H-Atome durch F, Cl und/oder Br ersetzt

sein können,
Cycloalkyl mit 3-8 C-Atomen oder
Cycloalkylalkylen mit 4-10 C-Atomen,

Hal F, Cl, Br oder I,
n 0, 1 oder 2

bedeuten,
sowie ihre pharmazeutisch verwendbaren Solvate, Salze, Tautomere und Stereoisomere, einschließlich deren Mischungen in allen Verhältnissen.

**12.** Verbindungen nach einem oder mehreren der Ansprüche 1-1 worin

$R^1$, $R^{1'}$ H,

$R^2$ H, OA oder Hal,
$R^3$ A, Ar, Het, $NR^4R^5$ oder $CHR^4R^5$,
wobei mindestens einer der Reste

$R^4$ oder $R^5$ $(CH_2)_nAr$ oder $(CH_2)_nHet$ bedeutet,

$R^4$, $R^5$ jeweils unabhängig voneinander H, A, $(CH_2)_nAr$ oder $(CH_2)_nHet$,
X fehlt, $CH_2$ oder NH,
Y fehlt,
Ar unsubstituiertes oder ein-, zwei- oder dreifach durch OH, OA, Hal und/oder A substituiertes Phenyl, Naphthyl oder 1,2,3,4-Tetrahydronaphthalin,
Het Pyridyl, Pyrimidinyl, Thienyl oder Furyl,
A jeweils unabhängig voneinander unverzweigtes oder verzweigtes Alkyl mit 1-10 C-Atomen,

worin 1-7 H-Atome durch F, Cl und/oder Br ersetzt

sein können,
Cycloalkyl mit 3-8 C-Atomen oder Cycloalkylalkylen mit 4-10 C-Atomen,
Hal F, Cl, Br oder I,
n 0, 1 oder 2

bedeuten,
sowie ihre pharmazeutisch verwendbaren Solvate, Salze, Tautomere und Stereoisomere, einschließlich deren Mischungen in allen Verhältnissen.

**13.** Verbindungen nach Anspruch 1, ausgewählt aus der Gruppe

| Verbindung Nr. | Struktur / Name |
|---|---|
| "A1" | 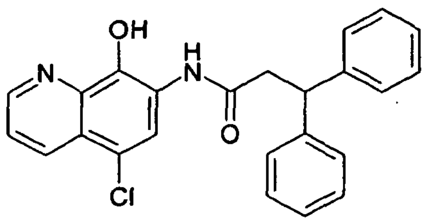 N-(5-Chlor-8-hydroxy-chinolin-7-yl)-3,3-diphenyl-propionamid |
| "A2" | 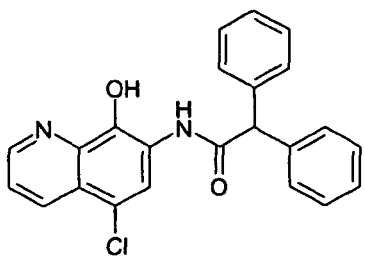 *N*-(5-Chlor-8-hydroxy-chinolin-7-yl)-2,2-diphenyl-acetamid |
| "A3" | 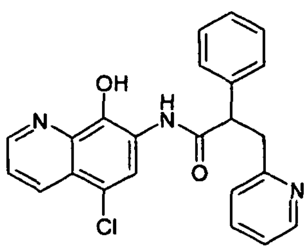 *N*-(5-Chlor-8-hydroxy-chinolin-7-yl)-2-phenyl-3-pyridin-2-yl-propionamid |

(fortgesetzt)

| Verbindung Nr. | Struktur / Name |
|---|---|
| "A4" | 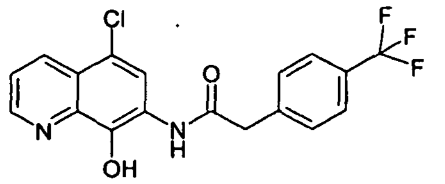 *N*-(5-Chlor-8-hydroxy-chinolin-7-yl)-2-(4-trifluormethyl-phenyl)-acetamid |
| "A5" | 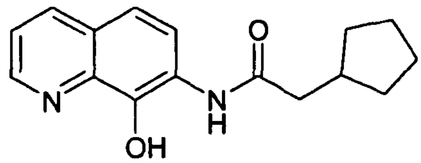 2-Cyclopentyl-*N*-(8-hydroxy-chinolin-7-yl)-acetamid |
| "A6" | 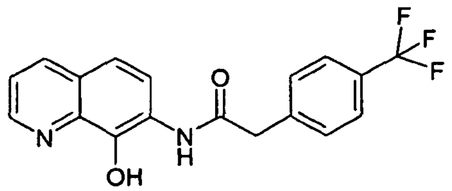 N-(8-Hydroxy-chinolin-7-yl)-2-(4-trifluormethyl-phenyl)-acetamid |
| "A7" | 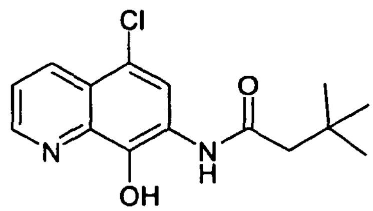 *N*-(5-Chlor-8-hydroxy-chinolin-7-yl)-3,3-dimethyl-butyramid |
| "A8" | 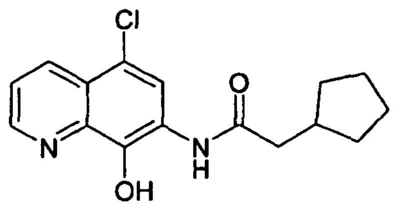 *N*-(5-Chlor-8-hydroxy-chinolin-7-yl)-2-cyclopentyl-acetamid |
| "A9" | 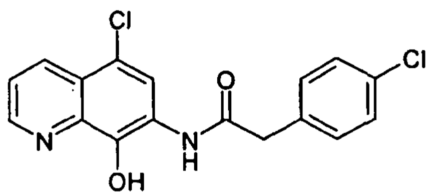 N-(5-Chlor-8-hydroxy-chinolin-7-yl)-2-(4-chlor-phenyl)-acetamid |
| "A10" | 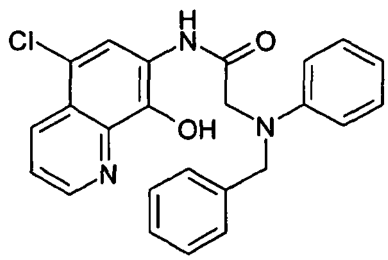 |

(fortgesetzt)

| Verbindung Nr. | Struktur / Name |
|---|---|
| | 2-(Benzyl-phenyl-amino)-*N*-(5-chlor-8-hydroxy-chinolin-7-yl)-acetamid |
| "A12" | 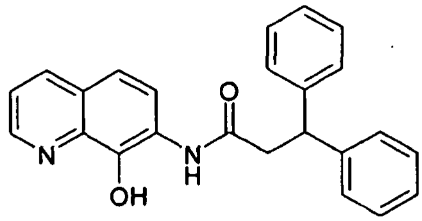 *N*-(8-Hydroxy-chinolin-7-yl)-3,3-diphenyl-propionamid |
| "A13" | 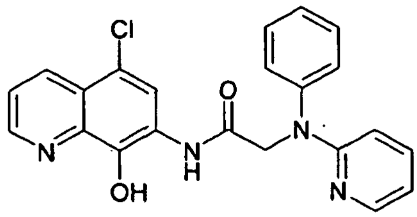 *N*-(5-Chlor-8-hydroxy-chinolin-7-yl)-2-(phenyl-pyridin-2-yl-amino)-acetamid |
| "A14" | 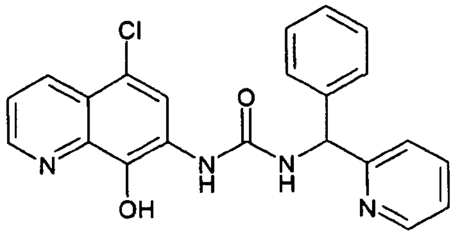 1-(5-Chlor-8-hydroxy-chinolin-7-yl)-3-(phenyl-pyridin-2-yl-methyl)-harnstoff |
| "A15" | 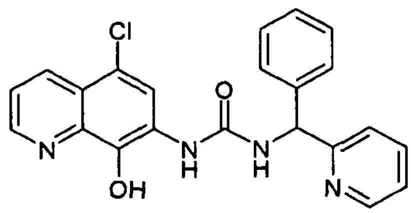 1-(5-Chlor-8-hydroxy-chinolin-7-yl)-3-(phenyl-pyridin-2-yl-methyl)-harnstoff |
| "A16" | 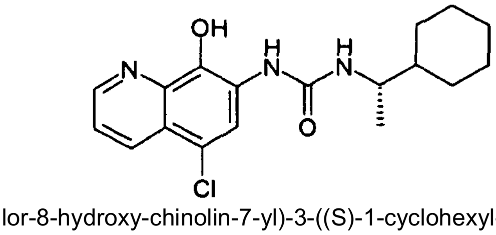 1-(5-Chlor-8-hydroxy-chinolin-7-yl)-3-((S)-1-cyclohexyl-ethyl)-harnstoff |
| "A17" | 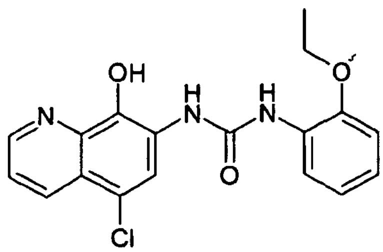 1-(5-Chlor-8-hydroxy-chinolin-7-yl)-3-(2-ethoxy-phenyl)-harnstoff |

(fortgesetzt)

| Verbindung Nr. | Struktur / Name |
|---|---|
| "A18" | 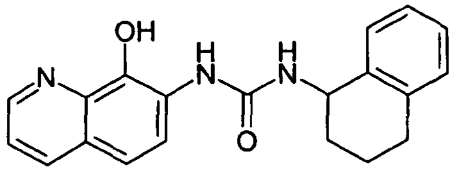 1-(8-Hydroxy-chinolin-7-yl)-3-(1,2,3,4-tetrahydro-naphthalin-1-yl)-harnstoff |
| "A19" | 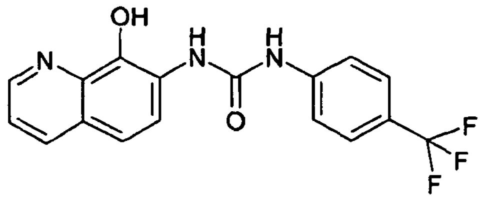 1-(8-Hydroxy-chinolin-7-yl)-3-(4-trifluormethyl-phenyl)-harnstoff |
| "A20" | 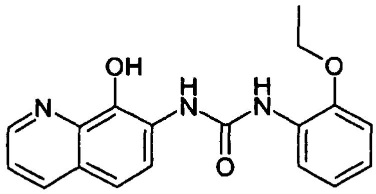 1-(2-Ethoxy-phenyl)-3-(8-hydroxy-chinolin-7-yl)-harnstoff |
| "A21" | 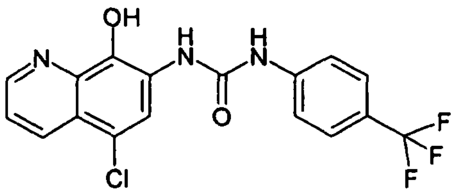 1-(5-Chlor-8-hydroxy-chinolin-7-yl)-3-(4-trifluormethyl-phenyl)-harnstoff |
| "A22" | 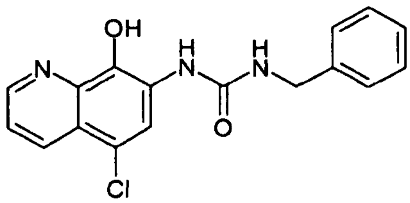 1-Benzyl-3-(5-chlor-8-hydroxy-chinolin-7-yl)-harnstoff |
| "A24" | 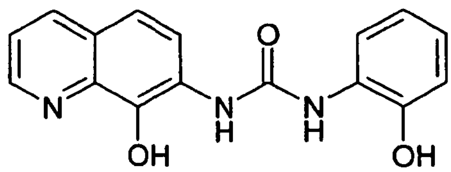 1-(2-Hydroxy-phenyl)-3-(8-hydroxy-chinolin-7-yl)-harnstoff |
| "A26" | 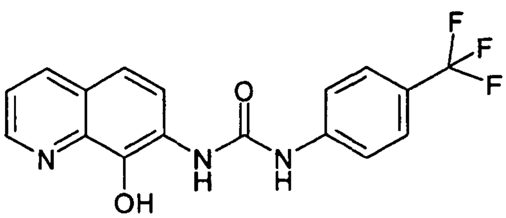 1-(2-Trifluormethyl-phenyl)-3-(8-hydroxy-chinolin-7-yl)-harnstoff |

sowie ihre pharmazeutisch verwendbaren Solvate, Salze, Tautomere und Stereoisomere, einschließlich deren Mischungen in allen Verhältnissen.

**14.** Verfahren zur Herstellung von Verbindungen der Formel I nach den Ansprüchen 1-13 sowie ihrer pharmazeutisch verwendbaren Salze, Solvate, Tautomeren und Stereoisomeren, **dadurch gekennzeichnet, daß** man

a) zur Herstellung von Verbindungen der Formel I, worin

X fehlt oder $CH_2$ bedeutet,

eine Verbindung der Formel II

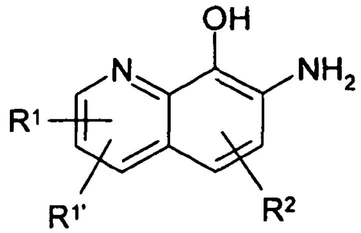

II

worin $R^1$, $R^{1'}$ und $R^2$ die in Anspruch 1 angegebenen Bedeutungen haben,
mit einer Verbindung der Formel III

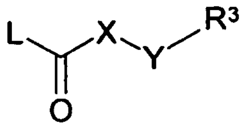

III ,

worin X fehlt oder $CH_2$ bedeutet,
Y und $R^3$ die in Anspruch 1 angegebenen Bedeutungen haben, und L Cl, Br, I oder eine freie oder reaktionsfähig funktionell abgewandelte OH-Gruppe bedeutet,
umsetzt,
oder
b) zur Herstellung von Verbindungen der Formel I, worin

X $NR^6$ bedeutet,

eine Verbindung der Formel II mit einer Verbindung der Formel IV

$O=C=N-Y-R^3$ IV

worin
Y und $R^3$ die in Anspruch 1 angebenen Bedeutungen haben, umsetzt,
oder
c) daß man sie aus einem ihrer funktionellen Derivate durch Behandeln mit einem solvolysierenden oder hydrogenolysierenden Mittel in Freiheit setzt,
oder
d) zur Herstellung von Verbindungen der Formel I, worin

X $NR^6$ oder O bedeutet,

eine Verbindung der Formel V

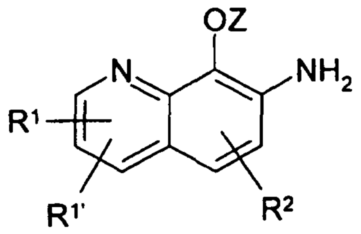

V

worin $R^1$, $R^{1'}$ und $R^2$ die in Anspruch 1 angegebenen Bedeutungen haben,
und Z eine Hydroxy-Schutzgruppe bedeutet,
mit einem Kupplungsreagenz ausgewählt aus der Gruppe

a) Isoproylidenchloroformiat,
b) p-Nitrophenylchloroformiat,
c) Diphosgen,
d) Triphosgen,

und einer Verbindung der Formel VI

$H\text{-}X\text{-}Y\text{-}R^3$ VI

worin X $NR^6$ oder O,
Y und $R^3$ die in Anspruch 1 angegebenen Bedeutungen haben,
umsetzt, und anschließend die Hydroxyschutzgruppe Z abspaltet,
und/oder
eine Base oder Säure der Formel I in eines ihrer Salze umwandelt.

**15.** Arzneimittel, enthaltend mindestens eine Verbindung der Formel I nach Anspruch 1-13 und/oder ihre pharmazeutisch verwendbaren Salze, Solvate, Tautomeren und Stereoisomeren, einschließlich deren Mischungen in allen Verhältnissen, sowie gegebenenfalls Träger- und/oder Hilfsstoffe.

**16.** Verwendung von Verbindungen nach Anspruch 1-13 sowie ihrer pharmazeutisch verwendbaren Salze, Solvate, Tautomeren und Stereoisomeren, einschließlich deren Mischungen in allen Verhältnissen, zur Herstellung eines Arzneimittels zur Behandlung von Tumoren, Tumorwachstum, Tumormetastasen und/oder AIDS.

**17.** Verwendung nach Anspruch 16, wobei der Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopfs und/oder der Lunge stammt.

**18.** Verwendung nach Anspruch 16, wobei der Tumor aus der Gruppe Monozytenleukämie, Lungenadenokarzinom, kleinzellige Lungenkarzinome, Bauchspeicheldrüsenkrebs, Kolonkarzinom, Glioblastome und/oder Brustkarzinom stammt.

**19.** Verwendung nach Anspruch 16, wobei es sich um einen Tumor des Blut- und Immunsystems handelt.

**20.** Verwendung nach Anspruch 16, wobei der Tumor aus der Gruppe der akuten myelotischen Leukämie, der chronischen myelotischen Leukämie, akuten lymphatischen Leukämie und/oder chronischen lymphatischen Leukämie stammt.

**21.** Verwendung von Verbindungen der Formel I gemäß Anspruch 1-13 und/oder ihrer physiologisch unbedenklichen Salze und Solvate zur Herstellung eines Arzneimittels zur Behandlung von Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

**22.** Verwendung von Verbindungen der Formel I gemäß Anspruch 1-13 und/oder ihrer physiologisch unbedenklichen Salze und Solvate zur Herstellung eines Arzneimittels zur Behandlung von Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

## Claims

**1.** Compounds of the formula I

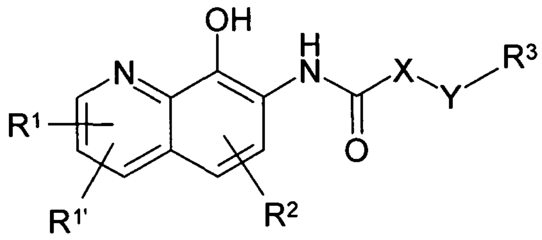

I

in which

$R^1$, $R^{1'}$, $R^2$ each, independently of one another, denote H, OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA or CN,
$R^3$ denotes A, Ar, Het, $NR^4R^5$ or $CHR^4R^5$, where at least one of the radicals $R^4$ or $R^5$ denotes $(CH_2)_nAr$ or $(CH_2)_nHet$,
$R^4$, $R^5$ each, independently of one another, denote H, A, $(CH_2)_nAr$ or $(CH_2)_nHet$,
X is absent or denotes $CH_2$, $NR^6$ or O,
Y is absent or denotes alkylene having 1-3 C atoms,
$R^6$ denotes H or A,
A, A' each, independently of one another, denote unbranched or branched alkyl having 1-10 C atoms,

in which 1-7 H atoms may be replaced by F, Cl

and/or Br,
cycloalkyl having 3-8 C atoms or
cycloalkylalkylene having 4-10 C atoms,
Hal denotes F, Cl, Br or I,
Ar denotes a saturated, unsaturated or aromatic carbocycle having 5-14 C atoms which is unsubstituted or mono-, di- or trisubstituted by OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA, COA, CHO or CN,
Het denotes a mono-, bi- or tricyclic saturated, unsaturated or aromatic heterocycle having 1 to 4 N, O and/or S atoms, which may be unsubstituted or mono-, di- or trisubstituted by OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA, CHO, COA, CN, =S, =NH, =NA and/or =O (carbonyl oxygen),
n denotes 0, 1 or 2,

and pharmaceutically usable solvates, salts, tautomers and stereoisomers thereof, including mixtures thereof in all ratios, where the following compounds are excluded:

N-(8-hydroxy-7-quinolyl)benzamide,
N-(8-hydroxy-7-quinolyl)octanamide,
2-(acetylamino)-N-(8-hydroxy-7-quinolyl)acetamide,
N-(8-hydroxy-7-quinolyl)-N'-phenylurea,
N-(8-hydroxy-7-quinolyl)-N'-octylurea,
N-(8-hydroxy-7-quinolyl)-N'-(phenylmethyl)urea,
N-(8-hydroxy-7-quinolyl)acetamide,

N-(5-chloro-8-hydroxy-7-quinolyl)-2-methylpropanamide,
4-[[[(5-chloro-8-hydroxy-7-quinolyl)amino]carbonyl]amino]-N,N-dioctylbenzenesulfonamide,
7-acetamido-8-hydroxy-5-quinolinecarboxylic acid,
7-acetamido-5-methyl-8-quinolinol.

**2.** Compounds according to Claim 1 in which

$R^1$, $R^{1'}$ denote H,

and pharmaceutically usable solvates, salts, tautomers and stereoisomers thereof, including mixtures thereof in all ratios.

**3.** Compounds according to Claim 1 or 2 in which

$R^2$ denotes H, OA or Hal,

and pharmaceutically usable solvates, salts, tautomers and stereoisomers thereof, including mixtures thereof in all ratios.

**4.** Compounds according to Claim 1, 2 or 3 in which

Ar denotes a saturated, unsaturated or aromatic carbocycle having 5-14 C atoms which is unsubstituted or mono-, di- or trisubstituted by OH, OA, Hal and/or A,

and pharmaceutically usable solvates, salts, tautomers and stereoisomers thereof, including mixtures thereof in all ratios.

**5.** Compounds according to one or more of Claims 1-4 in which

Het denotes a monocyclic aromatic heterocycle having 1 to 3 N, O and/or S atoms,

and pharmaceutically usable solvates, salts, tautomers and stereoisomers thereof, including mixtures thereof in all ratios.

**6.** Compounds according to one or more of Claims 1-5 in which

X is absent or denotes $CH_2$ or NH,

and pharmaceutically usable solvates, salts, tautomers and stereoisomers thereof, including mixtures thereof in all ratios.

**7.** Compounds according to one or more of Claims 1-6 in which

Y is absent,

and pharmaceutically usable solvates, salts, tautomers and stereoisomers thereof, including mixtures thereof in all ratios.

**8.** Compounds according to one or more of Claims 1-7 in which

Ar denotes phenyl, naphthyl or 1,2,3,4-tetrahydronaphthalene, each of which is unsubstituted or mono-, di- or trisubstituted by OH, OA, Hal and/or A,

and pharmaceutically usable solvates, salts, tautomers and stereoisomers thereof, including mixtures thereof in all ratios.

**9.** Compounds according to one or more of Claims 1-8 in which Het denotes pyridyl, pyrimidinyl, thienyl or furyl, and pharmaceutically usable solvates, salts, tautomers and stereoisomers thereof, including mixtures thereof in all ratios.

**10.** Compounds according to one or more of Claims 1-9 in which

$R^3$ denotes

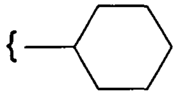 , 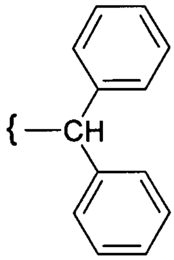 , 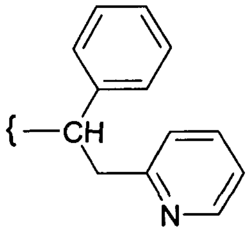 ,

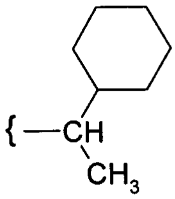 , 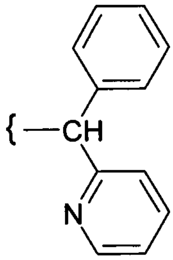 , 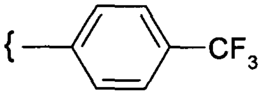 ,

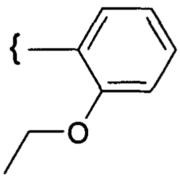 , 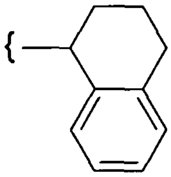 , 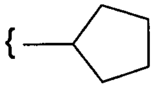 ,

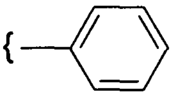 , 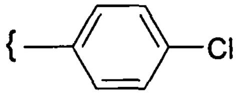 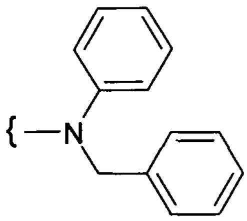 ,

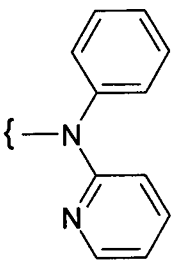 or 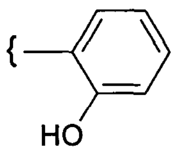 ,

and pharmaceutically usable solvates, salts, tautomers and stereoisomers thereof, including mixtures thereof in all ratios.

**11.** Compounds according to one or more of Claims 1-10 in which

$R^1$, $R^{1'}$ denote H,
$R^2$ denotes H, OA or Hal,
$R^3$ denotes A, Ar, Het, $NR^4R^5$ or $CHR^4R^5$, where at least one of the radicals $R^4$ or $R^5$ denotes $(CH_2)_nAr$ or $(CH_2)_nHet$,
$R^4$, $R^5$ each, independently of one another, denote H, A, $(CH_2)_nAr$ or $(CH_2)_nHet$,
X is absent or denotes $CH_2$ or NH,

Y is absent,
Ar denotes a saturated, unsaturated or aromatic carbocycle having 5-14 C atoms which is unsubstituted or mono-, di- or trisubstituted by OH, OA, Hal and/or A,
Het denotes a monocyclic aromatic heterocycle having 1 to 3 N, O and/or S atoms,
A in each case, independently of one another, denotes unbranched or branched alkyl having 1-10 C atoms,

in which 1-7 H atoms may be replaced by F, Cl and/or Br,

cycloalkyl having 3-8 C atoms or
cycloalkylalkylene having 4-10 C atoms,
Hal denotes F, Cl, Br or I,
n denotes 0, 1 or 2,

and pharmaceutically usable solvates, salts, tautomers and stereoisomers thereof, including mixtures thereof in all ratios.

**12.** Compounds according to one or more of Claims 1-11 in which

$R^1$, $R^{1'}$ denote H,
$R^2$ denotes H, OA or Hal,
$R^3$ denotes A, Ar, Het, $NR^4R^5$ or $CHR^4R^5$, where at least one of the radicals $R^4$ or $R^5$ denotes $(CH_2)_nAr$ or $(CH_2)_nHet$,
$R^4$, $R^5$ each, independently of one another, denote H, A, $(CH_2)_nAr$ or $(CH_2)_nHet$,
X is absent or denotes $CH_2$ or NH,
Y is absent,
Ar denotes phenyl, naphthyl or 1,2,3,4-tetrahydronaphthalene, each of which is unsubstituted or mono-, di- or trisubstituted by OH, OA, Hal and/or A,
Het denotes pyridyl, pyrimidinyl, thienyl or furyl,
A in each case, independently of one another, denotes unbranched or branched alkyl having 1-10 C atoms,

in which 1-7 H atoms may be replaced by F, Cl and/or Br,

cycloalkyl having 3-8 C atoms or
cycloalkylalkylene having 4-10 C atoms,

Hal denotes F, Cl, Br or I,
n denotes 0, 1 or 2,

and pharmaceutically usable solvates, salts, tautomers and stereoisomers thereof, including mixtures thereof in all ratios.

**13.** Compounds according to Claim 1, selected from the group

| Compound No. | Structure / name |
|---|---|
| "A1" | 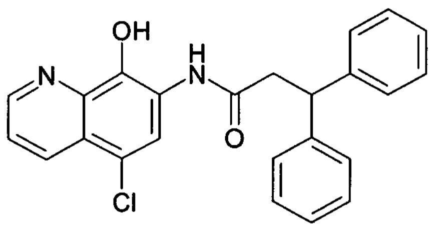 *N*-(5-Chloro-8-hydroxyquinolin-7-yl)-3,3-diphenylpropionamide |

(continued)

| Compound No. | Structure / name |
|---|---|
| "A2" | 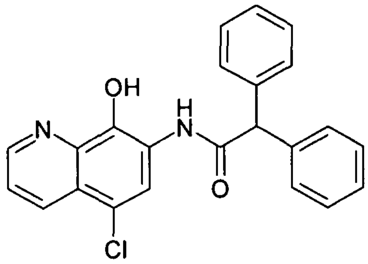 *N*-(5-Chloro-8-hydroxyquinolin-7-yl)-2,2-diphenylacetamide |
| "A3" | 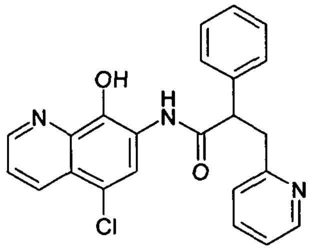 *N*-(5-Chloro-8-hydroxyquinolin-7-yl)-2-phenyl-3-pyridin-2-yl-propionamide |
| "A4" | 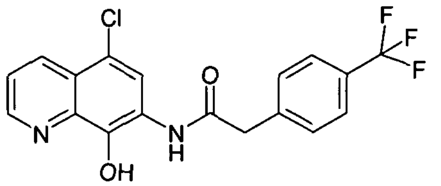 *N*-(5-Chloro-8-hydroxyquinolin-7-yl)-2-(4-trifluoromethyl-phenyl)acetamide |
| "A5" | 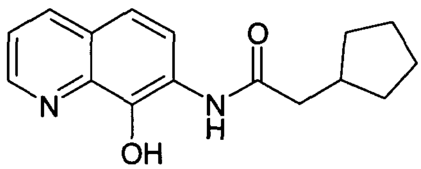 2-Cyclopentyl-*N*-(8-hydroxyquinolin-7-yl)acetamide |
| "A6" | 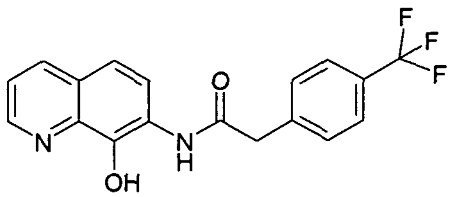 *N*-(8-Hydroxyquinolin-7-yl)-2-(4-trifluoromethylphenyl)-acetamide |
| "A7" | 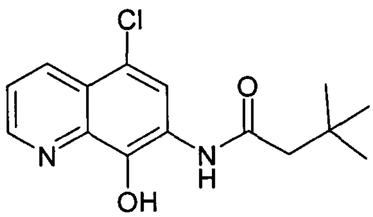 *N*-(5-Chloro-8-hydroxyquinolin-7-yl)-3,3-dimethylbutyramide |

(continued)

| Compound No. | Structure / name |
|---|---|
| "A8" | 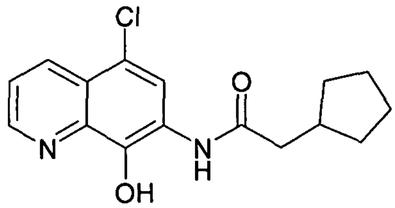 *N*-(5-Chloro-8-hydroxyquinolin-7-yl)-2-cyclopentylacetamide |
| "A9" | 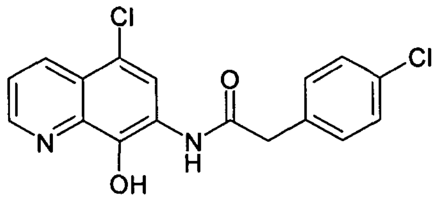 *N*-(5-Chloro-8-hydroxyquinolin-7-yl)-2-(4-chlorophenyl)-acetamide |
| "A10" | 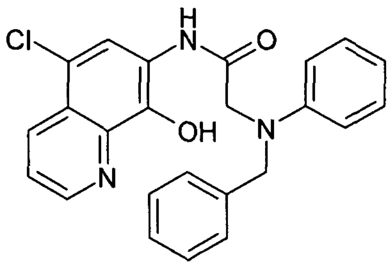 2-(Benzylphenylamino)-*N*-(5-chloro-8-hydroxyquinolin-7-yl)-acetamide |
| "A12" | 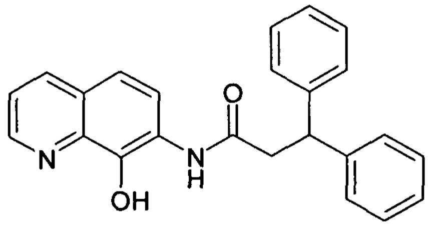 *N*-(8-Hydroxyquinolin-7-yl)-3,3-diphenylpropionamide |
| "A13" | 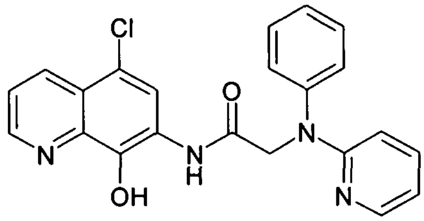 *N*-(5-Chloro-8-hydroxyquinolin-7-yl)-2-(phenylpyridin-2-yl-amino)acetamide |
| "A14" | 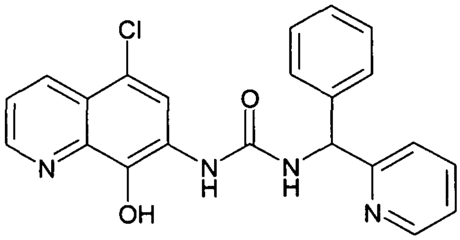 1-(5-Chloro-8-hydroxyquinolin-7-yl)-3-(phenylpyridin-2-yl-methyl)urea |

(continued)

| Compound No. | Structure / name |
|---|---|
| "A15" | 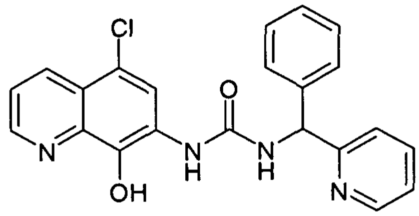 1-(5-Chloro-8-hydroxyquinolin-7-yl)-3-(phenylpyridin-2-yl-methyl)urea |
| "A16" | 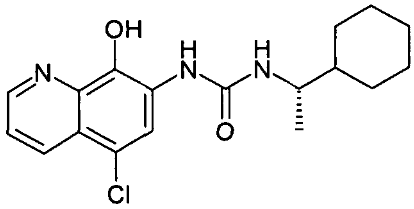 1-(5-Chloro-8-hydroxyquinolin-7-yl)-3-((S)-1-cyclohexyl-ethyl)urea |
| "A17" | 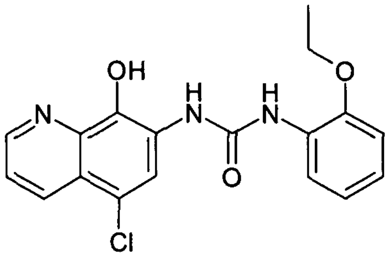 1-(5-Chloro-8-hydroxyquinolin-7-yl)-3-(2-ethoxyphenyl)urea |
| "A18" | 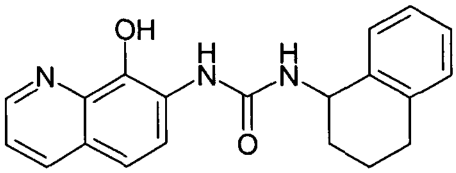 1-(8-Hydroxyquinolin-7-yl)-3-(1,2,3,4-tetrahydronaphthalen-1-yl)urea |
| "A19" | 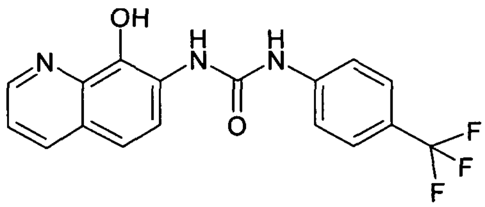 1-(8-Hydroxyquinolin-7-yl)-3-(4-trifluoromethylphenyl)urea |
| "A20" | 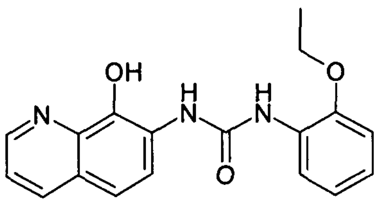 1-(2-Ethoxyphenyl)-3-(8-hydroxyquinolin-7-yl)urea |

(continued)

| Compound No. | Structure / name |
|---|---|
| "A21" | 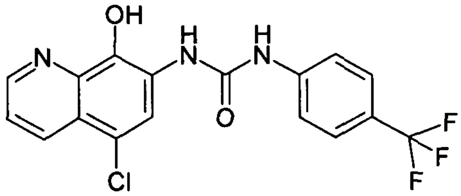 1-(5-Chloro-8-hyd roxyq u inolin-7-yl)-3-(4-trifluoromethyl-phenyl)urea |
| "A22" | 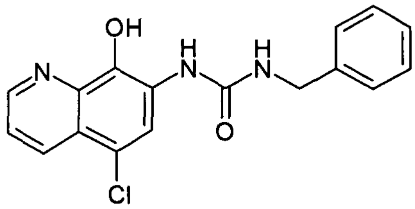 1-Benzyl-3-(5-chloro-8-hydroxyquinolin-7-yl)urea |
| "A24" | 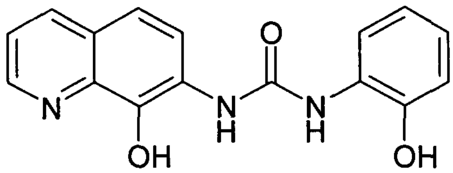 1-(2-Hydroxyphenyl)-3-(8-hydroxyquinolin-7-yl)urea |
| "A26" | 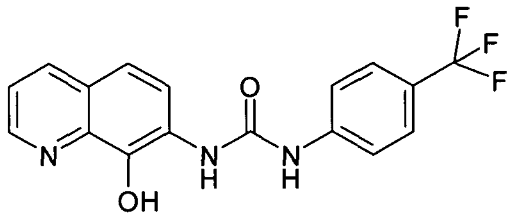 1-(2-Trifluoromethylphenyl)-3-(8-hydroxyquinolin-7-yl)urea |

and pharmaceutically usable solvates, salts, tautomers and stereoisomers thereof, including mixtures thereof in all ratios.

**14.** Process for the preparation of compounds of the formula I according to Claims 1-13 and pharmaceutically usable salts, solvates, tautomers and stereoisomers thereof, **characterised in that**

a) for the preparation of compounds of the formula I in which

X is absent or denotes $CH_2$,

a compound of the formula II

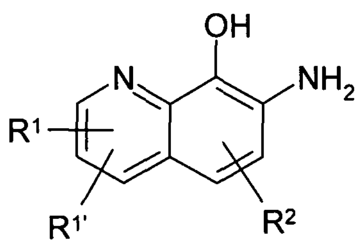

II

in which $R^1$, $R^{1'}$ and $R^2$ have the meanings indicated in Claim 1,

is reacted with a compound of the formula III

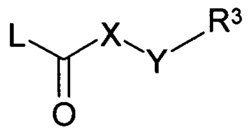

III ,

in which X is absent or denotes $CH_2$,
Y and $R^3$ have the meanings indicated in Claim 1,
and L denotes Cl, Br, I or a free or reactively functionally modified OH group,
or
b) for the preparation of compounds of the formula I in which

X denotes $NR^6$,
a compound of the formula II is reacted with a compound of the formula IV

$O{=}C{=}N\text{-}Y\text{-}R^3$ IV

in which
Y and $R^3$ have the meanings indicated in Claim 1,
or

c) **in that** they are liberated from one of their functional derivatives by treatment with a solvolysing or hydrogenolysing agent,
or
d) for the preparation of compounds of the formula I in which

X denotes $NR^6$ or O,

a compound of the formula V

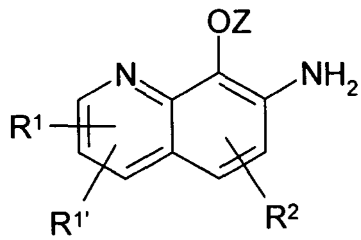

V

in which $R^1$, $R^{1'}$ and $R^2$ have the meanings indicated in Claim 1, and Z denotes a hydroxyl-protecting group, is reacted with a coupling reagent selected from the group

a) isoproylidene chloroformate,
b) p-nitrophenyl chloroformate,
c) diphosgene,
d) triphosgene,

and a compound of the formula VI

$H\text{-}X\text{-}Y\text{-}R^3$ VI

in which X denotes $NR^6$ or O,
Y and $R^3$ have the meanings indicated in Claim 1,
and the hydroxyl-protecting group Z is subsequently cleaved off, and/or
a base or acid of the formula I is converted into one of its salts.

**15.** Medicament comprising at least one compound of the formula I according to Claims 1-13 and/or pharmaceutically usable salts, solvates, tautomers and stereoisomers thereof, including mixtures thereof in all ratios, and optionally excipients and/or adjuvants.

**16.** Use of compounds according to Claims 1-13 and pharmaceutically usable salts, solvates, tautomers and stereoisomers thereof, including mixtures thereof in all ratios, for the preparation of a medicament for the treatment of tumours, tumour growth, tumour metastases and/or AIDS.

**17.** Use according to Claim 16, where the tumour originates from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

**18.** Use according to Claim 16, where the tumour originates from the group monocytic leukaemia, lung adenocarcinoma, small-cell lung carcinomas, pancreatic cancer, colon carcinoma, glioblastomas and/or breast carcinoma.

**19.** Use according to Claim 16, where the tumour is a tumour of the blood and immune system.

**20.** Use according to Claim 16, where the tumour originates from the group of acute myeloid leukaemia, chronic myeloid leukaemia, acute lymphatic leukaemia and/or chronic lymphatic leukaemia.

**21.** Use of compounds of the formula I according to Claims 1-13 and/or physiologically acceptable salts and solvates thereof for the preparation of a medicament for the treatment of tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

**22.** Use of compounds of the formula I according to Claims 1-13 and/or physiologically acceptable salts and solvates thereof for the preparation of a medicament for the treatment of tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitors.

## Revendications

**1.** Composés de formule I

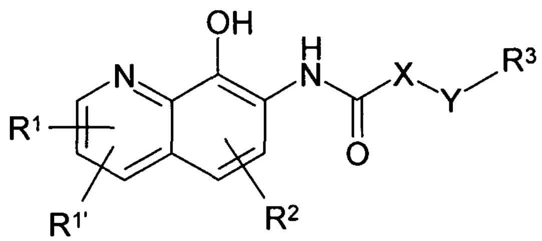

I

dans laquelle

$R^1$, $R^{1'}$, $R^2$ désignent chacun, indépendamment l'un de l'autre, H, OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA ou CN,
$R^3$ désigne A, Ar, Het, $NR^4R^5$ ou $CHR^4R^5$, où au moins l'un des radicaux $R^4$ ou $R^5$ désigne $(CH_2)_nAr$ ou $(CH_2)_nHet$,
$R^4$, $R^5$ désignent chacun, indépendamment l'un de l'autre, H, A, $(CH_2)_nAr$ ou $(CH_2)_nHet$,
X est absent ou désigne $CH_2$, $NR^6$ ou O,

Y est absent ou désigne alkylène ayant de 1 à 3 atomes de C,
$R^6$ désigne H ou A,
A, A' désignent chacun, indépendamment l'un de l'autre, alkyle non ramifié ou ramifié ayant de 1 à 10 atomes de C,

où 1-7 atomes de H peuvent être remplacés par F, Cl et/ou Br,
cycloalkyle ayant de 3-8 atomes de C ou
cycloalkylalkylène ayant de 4-10 atomes de C,

Hal désigne F, Cl, Br ou I,
Ar désigne un carbocycle saturé, insaturé ou aromatique, ayant 5-14 atomes de C, qui est non substitué ou mono-, di- ou trisubstitué par OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA, COA, CHO ou CN,
Het désigne un hétérocycle mono-, bi- ou tricyclique saturé, insaturé ou aromatique, ayant de 1 à 4 atomes de N, O et/ou S, qui peut être non substitué ou mono-, di- ou trisubstitué par OH, OA, SH, SA, SOA, $SO_2A$, Hal, $NO_2$, $NH_2$, NHA, NAA', A, $SO_2NH_2$, $SO_2NHA$, $SO_2NAA'$, $CONH_2$, CONHA, CONAA', NACOA', $NASO_2A'$, COOH, COOA, CHO, COA, CN, =S, =NH, =NA et/ou =O (oxygène de carbonyle),
n désigne 0, 1 ou 2,

et les solvats, sels, tautomères et stéréoisomères pharmaceutiquement utilisables de ceux-ci, y compris des mélanges de ceux-ci en toutes proportions,
où les composés qui suivent sont exclus:

N-(8-hydroxy-7-quinolyl)benzamide,
N-(8-hydroxy-7-quinolyl)octanamide,
2-(acétylamino)-N-(8-hydroxy-7-quinolyl)acétamide,
N-(8-hydroxy-7-quinolyl)-N'-phénylurée,
N-(8-hydroxy-7-quinolyl)-N'-octylurée,
N-(8-hydroxy-7-quinolyl)-N'-(phénylméthyl)urée,
N-(8-hydroxy-7-quinolyl)acétamide,
N-(5-chloro-8-hydroxy-7-quinolyl)-2-méthylpropanamide,
4-[[[(5-chloro-8-hydroxy-7-quinolyl)amino]carbonyl]amino]-N,N-dioctylbenzènesulfonamide,
acide 7-acétamido-8-hydroxy-5-quinolinecarboxylique,
7-acétamido-5-méthyl-8-quinolinol.

**2.** Composés selon la revendication 1, dans lesquels

$R^1$, $R^{1'}$ désignent H,

et les solvats, sels, tautomères et stéréoisomères pharmaceutiquement utilisables de ceux-ci, y compris des mélanges de ceux-ci en toutes proportions.

**3.** Composés selon la revendication 1 ou 2, dans lesquels

$R^2$ désigne H, OA ou Hal,

et les solvats, sels, tautomères et stéréoisomères pharmaceutiquement utilisables de ceux-ci, y compris des mélanges de ceux-ci en toutes proportions.

**4.** Composés selon la revendication 1, 2 ou 3, dans lesquels

Ar désigne un carbocycle saturé, insaturé ou aromatique, ayant 5-14 atomes de C, qui est non substitué ou mono-, di- ou trisubstitué par OH, OA, Hal et/ou A,

et les solvats, sels, tautomères et stéréoisomères pharmaceutiquement utilisables de ceux-ci, y compris des mélanges de ceux-ci en toutes proportions.

**5.** Composés selon l'une ou plusieurs parmi les revendications 1-4, dans lesquels

Het désigne un hétérocycle monocyclique aromatique, ayant de 1 à 3 atomes de N, O et/ou S,

et les solvats, sels, tautomères et stéréoisomères pharmaceutiquement utilisables de ceux-ci, y compris des mélanges de ceux-ci en toutes proportions.

**6.** Composés selon l'une ou plusieurs parmi les revendications 1-5, dans lesquels

X est absent ou désigne $CH_2$ ou NH,

et les solvats, sels, tautomères et stéréoisomères pharmaceutiquement utilisables de ceux-ci, y compris des mélanges de ceux-ci en toutes proportions.

**7.** Composés selon l'une ou plusieurs parmi les revendications 1-6, dans lesquels

Y est absent,

et les solvats, sels, tautomères et stéréoisomères pharmaceutiquement utilisables de ceux-ci, y compris des mélanges de ceux-ci en toutes proportions.

**8.** Composés selon l'une ou plusieurs parmi les revendications 1-7, dans lesquels

Ar désigne phényle, naphtyle ou 1,2,3,4-tetrahydronaphtalène, don't chacun est non substitué ou mono-, di- ou trisubstitué par OH, OA, Hal et/ou A,

et les solvats, sels, tautomères et stéréoisomères pharmaceutiquement utilisables de ceux-ci, y compris des mélanges de ceux-ci en toutes proportions.

**9.** Composés selon l'une ou plusieurs parmi les revendications 1-8, dans lesquels

Het désigne pyridyle, pyrimidinyle, thiényle ou furyle,

et les solvats, sels, tautomères et stéréoisomères pharmaceutiquement utilisables de ceux-ci, y compris des mélanges de ceux-ci en toutes proportions.

**10.** Composés selon l'une ou plusieurs parmi les revendications 1-9, dans lesquels

$R^3$ désigne

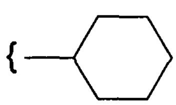 , 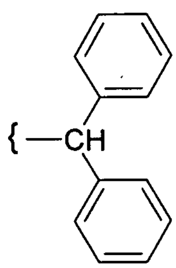 , 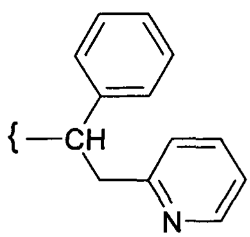 ,

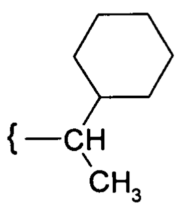 , 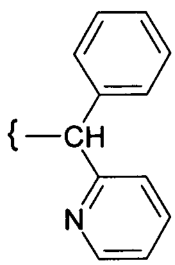 , 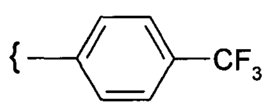 ,

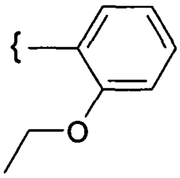 , 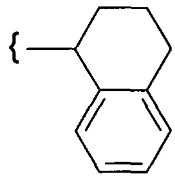 , 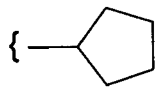 ,

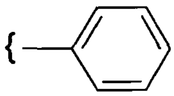 , 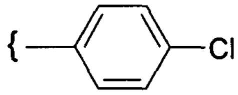 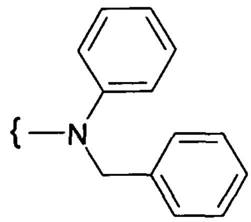 ,

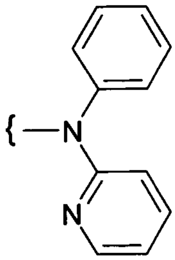 ou 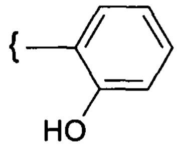 ,

et les solvats, sels, tautomères et stéréoisomères pharmaceutiquement utilisables de ceux-ci, y compris des mélanges de ceux-ci en toutes proportions.

**11.** Composés selon l'une ou plusieurs parmi les revendications 1-10, dans lesquels

$R^1$, $R^{1'}$ désigne H,
$R^2$ désigne H, OA ou Hal,
$R^3$ désigne A, Ar, Het, $NR^4R^5$ ou $CHR^4R^5$, où au moins l'un des radicaux $R^4$ ou $R^5$ désigne $(CH_2)_nAr$ ou $(CH_2)_nHet$,
$R^4$, $R^5$ désignent chacun, indépendamment l'un de l'autre, H, A, $(CH_2)_nAr$ ou $(CH_2)_nHet$,
X est absent ou désigne $CH_2$ ou NH,
Y est absent,
Ar désigne un carbocycle saturé, insaturé ou aromatique, ayant 5-14 atomes de C, qui est non substitué ou mono-, di- ou trisubstitué par OH, OA, Hal et/ou A,
Het désigne un hétérocycle monocyclique aromatique, ayant de 1 à 3 atomes de N, O et/ou S,
A désignent chacun, indépendamment l'un de l'autre, alkyle non ramifié ou ramifié ayant de 1 à 10 atomes de C,

où 1-7 atomes de H peuvent être remplacés par F, Cl et/ou Br,

cycloalkyle ayant de 3-8 atomes de C ou
cycloalkylalkylène ayant de 4-10 atomes de C,
Hal désigne F, Cl, Br ou I,
n désigne 0, 1 ou 2,

et les solvats, sels, tautomères et stéréoisomères pharmaceutiquement utilisables de ceux-ci, y compris des mélanges de ceux-ci en toutes proportions.

**12.** Composés selon l'une ou plusieurs parmi les revendications 1-11, dans lesquels

$R^1$, $R^{1'}$ désigne H,
$R^2$ désigne H, OA ou Hal,
$R^3$ désigne A, Ar, Het, $NR^4R^5$ ou $CHR^4R^5$, où au moins l'un des radicaux $R^4$ ou $R^5$ désigne $(CH_2)_nAr$ ou $(CH_2)_nHet$,
$R^4$, $R^5$ désignent chacun, indépendamment l'un de l'autre, H, A, $(CH_2)_nAr$ ou $(CH_2)_nHet$,
X est absent ou désigne $CH_2$ ou NH,

Y est absent,
Ar désigne phényle, naphtyle ou 1,2,3,4-tetrahydronaphtalène, don't chacun est non substitué ou mono-, di- ou trisubstitué par OH, OA, Hal et/ou A,
Het désigne pyridyle, pyrimidinyle, thiényle ou furyle,
A désignent chacun, indépendamment l'un de l'autre, alkyle non ramifié ou ramifié ayant de 1 à 10 atomes de C,

où 1-7 atomes de H peuvent être remplacés par F, Cl et/ou Br,

cycloalkyle ayant de 3-8 atomes de C ou
cycloalkylalkylène ayant de 4-10 atomes de C,

Hal désigne F, Cl, Br ou I,
n désigne 0, 1 ou 2,

et les solvats, sels, tautomères et stéréoisomères pharmaceutiquement utilisables de ceux-ci, y compris des mélanges de ceux-ci en toutes proportions.

**13.** Composés selon la revendication 1, choisis dans le groupe constitué par

| Composé N°. | Structure / nom |
|---|---|
| "A1" | 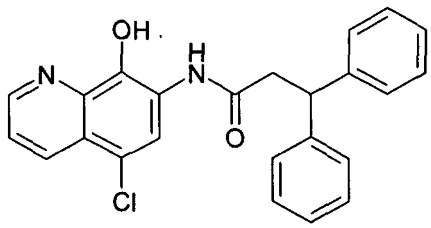 *N*-(5-Chloro-8-hydroxyquinolin-7-yl)-3,3-diphénylpropionamide |
| "A2" | 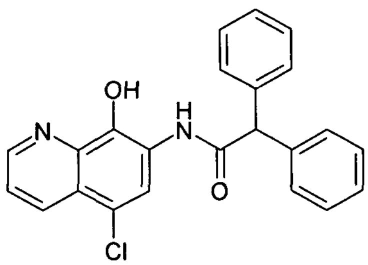 *N*-(5-Chloro-8-hydroxyquinolin-7-yl)-2,2-diphénylacétamide |
| "A3" | 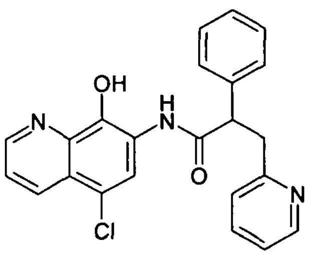 *N*-(5-Chloro-8-hydroxyquinolin-7-yl)-2-phényl-3-pyridin-2-yl-propionamide |
| "A4" | 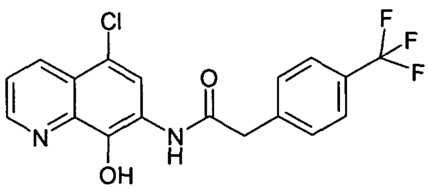 *N*-(5-Chloro-8-hydroxyquinolin-7-yl)-2-(4-trifluorométhyl-phényl)acétamide |

(suite)

| Composé N°. | Structure / nom |
|---|---|
| "A5" | 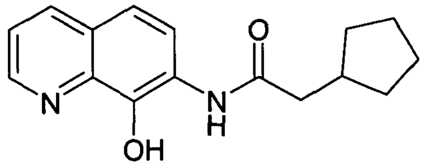 2-Cyclopentyl-*N*-(8-hydroxyquinolin-7-yl)acétamide |
| "A6" | 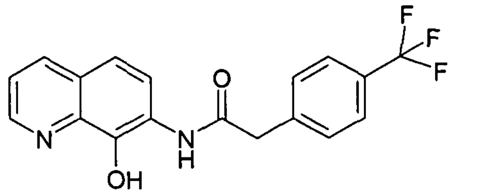 *N*-(8-Hydroxyquinolin-7-yl)-2-(4-trifluorométhylphényl)-acétamide |
| "A7" | 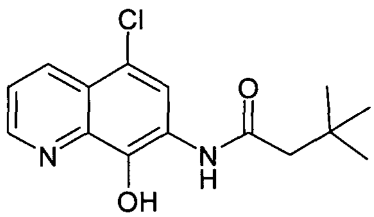 *N*-(5-Chloro-8-hydroxyquinolin-7-yl)-3,3-diméthylbutyramide |
| "A8" | 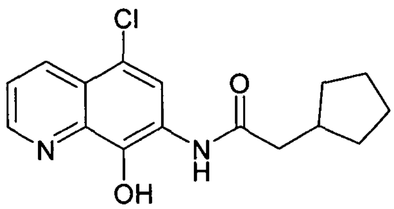 *N*-(5-Chloro-8-hydroxyquinolin-7-yl)-2-cyclopentylacétamide |
| "A9" | 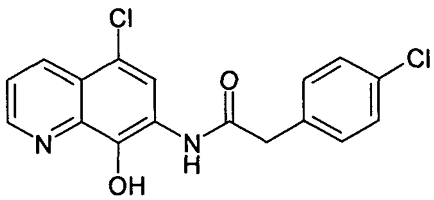 *N*-(5-Chloro-8-hydroxyquinolin-7-yl)-2-(4-chlorophényl)-acétamide |
| "A10" | 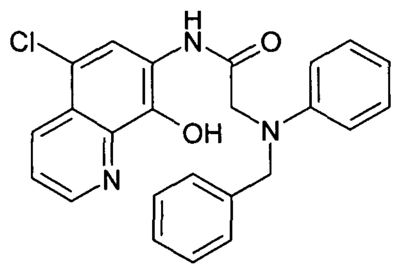 2-(Benzylphénylamino)-*N*-(5-chloro-8-hydroxyquinolin-7-yl)-acétamide |

(suite)

| Composé N°. | Structure / nom |
|---|---|
| "A12" | 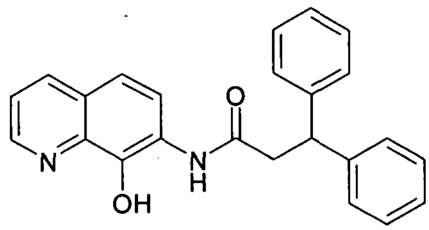<br>*N*-(8-Hydroxyquinolin-7-yl)-3,3-diphénylpropionamide |
| "A13" | 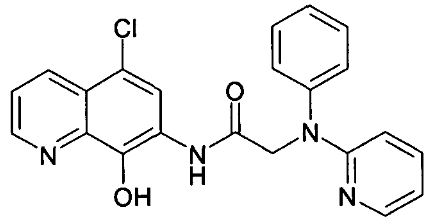<br>*N*-(5-Chloro-8-hydroxyquinolin-7-yl)-2-(phénylpyridin-2-yl-amino)acétamide |
| "A14" | 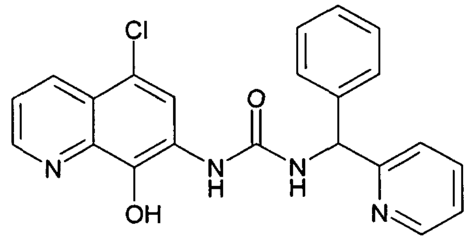<br>1-(5-Chloro-8-hydroxyquinolin-7-yl)-3-(phénylpyridin-2-yl-méthyl)urée |
| "A15" | 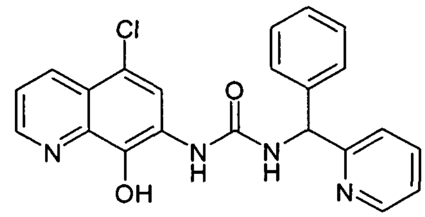<br>1-(5-Chloro-8-hydroxyquinolin-7-yl)-3-(phénylpyridin-2-yl-méthyl)urée |
| "A16" | 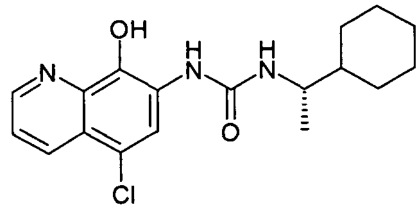<br>1-(5-Chloro-8-hydroxyquinolin-7-yl)-3-((S)-1-cyclohexyl-éthyl)urée |
| "A17" | 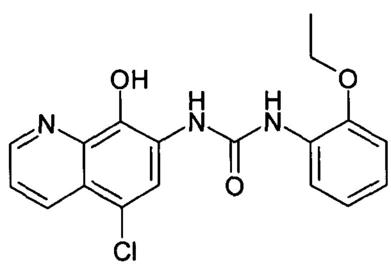<br>1-(5-Chloro-8-hydroxyquinolin-7-yl)-3-(2-éthoxyphényl)urée |

(suite)

| Composé N°. | Structure / nom |
|---|---|
| "A18" | 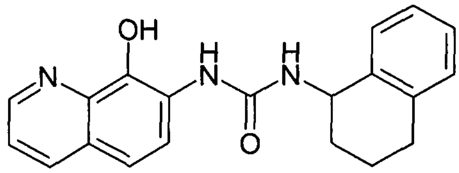 1-(8-Hydroxyquinolin-7-yl)-3-(1,2,3,4-tetrahydronaphtalèn-1-yl)urée |
| "A19" | 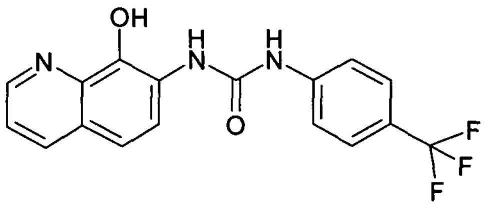 1-(8-Hydroxyquinolin-7-yl)-3-(4-trifluorométhylphényl)urée |
| "A20" | 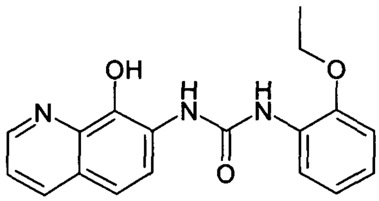 1-(2-Éthoxyphényl)-3-(8-hydroxyquinolin-7-yl)urée |
| "A21" | 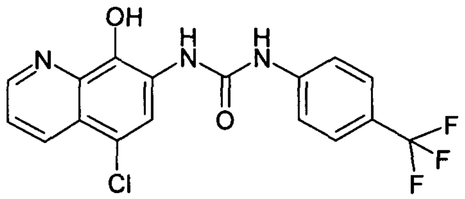 1-(5-Chloro-8-hydroxyquinolin-7-yl)-3-(4-trifluorométhyl-phényl)urée |
| "A22" | 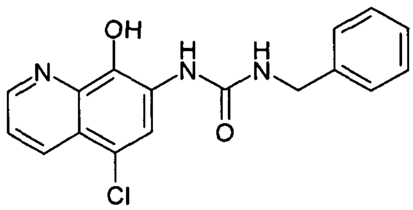 1-Benzyl-3-(5-chloro-8-hydroxyquinolin-7-yl)urée |
| "A24" | 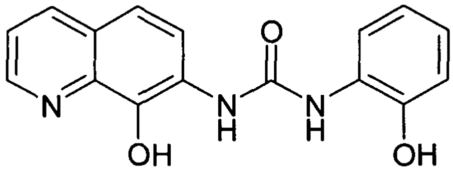 1-(2-Hydroxyphényl)-3-(8-hydroxyquinolin-7-yl)urée |
| "A26" | 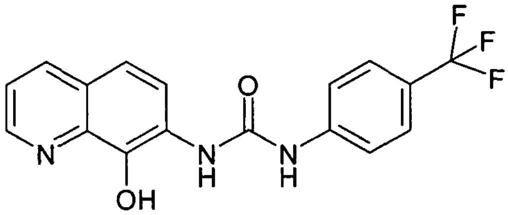 1-(2-Trifluorométhylphényl)-3-(8-hydroxyquinolin-7-yl)urée |

et les solvats, sels, tautomères et stéréoisomères pharmaceutiquement utilisables de ceux-ci, y compris des mélanges de ceux-ci en toutes proportions.

**14.** Procédé de préparation de composés de formule I selon les revendications 1-13 et de sels, solvats, tautomères et stéréoisomères pharmaceutiquement utilisables de ceux-ci, **caractérisé en ce que**

a) pour la préparation d'un composé de formule I dans laquelle

X est absent ou désigne $CH_2$,

un composé de formule II

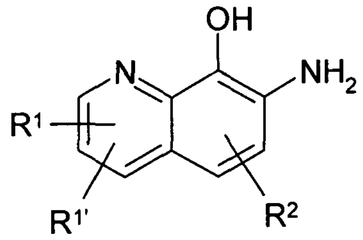

II

dans laquelle $R^1$, $R^{1'}$ et $R^2$ revêtent les significations indiquées selon la revendication 1,
est réagi avec un composé de formule III

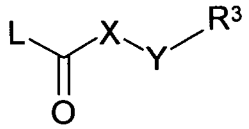

III ,

dans laquelle X est absent ou désigne $CH_2$,
Y et $R^3$ revêtent les significations indiquées selon la revendication 1, et L désigne Cl, Br, I ou un groupement OH libre ou modifié de manière fonctionnelle et réactive,
ou
b) pour la préparation d'un composé de formule I dans laquelle

X désigne $NR^6$,

un composé de formule II est réagi avec un composé de formule IV

$O=C=N-Y-R^3$ IV

dans laquelle
Y et $R^3$ revêtent les significations indiquées selon la revendication 1,
ou
c) **en ce qu'**ils sont libérés à partir de l'un de leurs dérivés fonctionnels par traitement par un agent de solvolyse ou d'hydrogénolyse,
ou
d) pour la préparation d'un composé de formule I dans laquelle

X désigne $NR^6$ ou O,

un composé de formule V

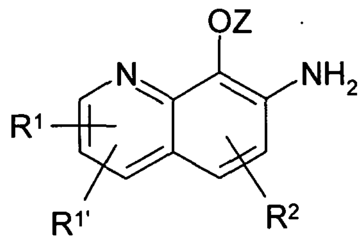

V

dans laquelle $R^1$, $R^{1'}$ et $R^2$ revêtent les significations indiquées selon la revendication 1, et Z désigne un groupe de protection d'hydroxyle, est réagi avec un réactif de couplage choisi dans le groupe constitué par

a) le chloroformiate d'isoproylidène,
b) le chloroformiate de p-nitrophényle,
c) le diphosgène,
d) le triphosgène,

et un composé de formule VI

$H\text{-}X\text{-}Y\text{-}R^3$ VI

dans laquelle X désigne $NR^6$ ou O, Y et $R^3$ revêtent les significations indiquées selon la revendication 1, et ensuite le groupe de protection de hydroxyle Z est enlevé par séparation, et/ou une base ou un acide de formule I est converti(e) en l'un de ses sels.

**15.** Médicaments comprenant au moins un composé de formule I selon les revendications 1-13 et/ou des sels, solvats, tautomères et stéréoisomères pharmaceutiquement utilisables de celui-ci, y compris des mélanges de ceux-ci en toutes proportions, et éventuellement des excipients et/ou des adjuvants.

**16.** Utilisation de composés selon les revendications 1-13 et de sels, solvats, tautomères et stéréoisomères pharmaceutiquement utilisables de ceux-ci, y compris des mélanges de ceux-ci en toutes proportions, pour la préparation d'un médicament destiné au traitement de tumeurs, la croissance tumorale, les métastases tumorales et/ou SIDA.

**17.** Utilisation selon la revendication 16, dans laquelle la tumeur provient du groupe constitué par les tumeurs de l'épithélium squameux, la vessie, l'estomac, le rein, la tête et le cou, l'oesophage, le col de l'utérus, la thyroïde, les intestins, le foie, le cerveau, la prostate, le tractus urogénital, le système lymphatique, l'estomac, le larynx et/ou le poumon.

**18.** Utilisation selon la revendication 16, dans laquelle la tumeur provient du groupe constitué par la leucémie monocytique, l'adénocarcinome du poumon, les carcinomes du poumon à petites cellules, le cancer du pancréas, les glioblastomes, le carcinome du côlon et/ou le carcinome du sein.

**19.** Utilisation selon la revendication 16, dans laquelle la tumeur est une tumeur du système sanguin et immunitaire.

**20.** Utilisation selon la revendication 16, dans laquelle la tumeur provient du groupe constitué par la leucémie myéloïde aiguë, la leucémie myéloïde chronique, la leucémie lymphoïde aiguë et/ou la leucémie lymphoïde chronique.

**21.** Utilisation de composés de formule I selon les revendications 1-13 et/ou de sels et de solvats physiologiquement acceptables de ceux-ci pour la préparation d'un médicament destiné au traitement de tumeurs, dans laquelle une quantité thérapeutiquement efficace d'un composé de formule I est administrée en association avec un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

**22.** Utilisation de composés de formule I selon les revendications 1-13 et/ou de sels et de solvats physiologiquement acceptables de ceux-ci pour la préparation d'un médicament destiné au traitement de tumeurs, dans laquelle une quantité thérapeutiquement efficace d'un composé de formule I est administrée en association avec une radiothérapie et un composé issu du groupe constitué par 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) d'autres inhibiteurs de l'angiogenèse.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6030983 A
- US 5712289 A
- US 5646150 A
- WO 9429308 A
- WO 0050032 A
- US 6069134 A